# FASCICULE DE BREVET EUROPEEN

(11) **EP 2 736 639 B1**
(45) Date de publication et mention de la délivrance du brevet: **10.02.2016**
(21) Numéro de dépôt: 12744091.5
(22) Date de dépôt: 12.07.2012
(51) Int. Cl.: B67D 7/44, C12M 1/12, B01L 1/02

(54) **DISPOSITIF DE JONCTION ETANCHE POUR LE TRANSFERT ASEPTIQUE D'UN PRODUIT BIOPHARMACEUTIQUE**
DICHTES VERBINDUNGSSTÜCK ZUM ASEPTISCEN TRANSFER VON BIOPHARMAZEUTISCHEN PRODUKTEN
LEAKPROOF JUNCTION DEVICE FOR ASEPTIC TRANSFER OF BIOPHARMACEUTIC PRODUCTS

(30) Priorité: 29.07.2011 FR 1157007
(43) Date de publication de la demande: 04.06.2014
(73) Titulaire: SARTORIUS STEDIM FMT SAS, 13400 Aubagne (FR)
(72) Inventeur: NODIN, Gaëlle, F-83470 Saint Maximin La Sainte Baume (FR); GIBELIN, Jérémy, F-83330 Le Beausset (FR)
(74) Mandataire: Derambure Conseil
(86) Numéro de dépôt international: PCT/FR2012/051664
(87) Numéro de publication internationale: WO 2013/017765

(56) Documents cités:
- WO-A1-2010/054031
- FR-A1- 2 872 446
- GB-A- 2 102 719
- GB-A- 2 218 663

## Description

L'invention est relative au domaine du transfert aseptique de produits biopharmaceutiques entre un conteneur et une enceinte close.

Plus précisément, l'invention concerne, selon un premier aspect, un dispositif de jonction étanche spécialement destiné à assurer le transfert aseptique de produits appartenant au domaine biopharmaceutique entre un conteneur et une enceinte close. L'invention concerne également, selon un deuxième aspect, un ensemble comprenant un conteneur, un tel dispositif de jonction étanche et une enceinte en vue d'assurer le transfert aseptique de produits biopharmaceutiques entre le conteneur et l'enceinte close. L'invention concerne en outre, selon un troisième aspect, un procédé de transfert aseptique d'un produit biopharmaceutique entre le conteneur et l'enceinte close.

Il convient d'entendre ici par produit biopharmaceutique ou produit de biopharmacie, ce qui est relatif à la biotechnologie, à la pharmacie et plus généralement au domaine médical. En particulier, un produit biopharmaceutique est un produit issu de la biotechnologie - milieux de cultures, cultures cellulaires, solutions tampon, liquides de nutrition artificielle - ou un produit destiné à être utilisé dans le domaine pharmaceutique ou médical, au moins pour partie, sous forme solide plus ou moins finement divisé, sous forme liquide, ou sous forme pâteuse ou encore, plus généralement, un produit matériel - bouchon, récipient, tube ou ports intégrés, seringue, piston de seringue, moyen fonctionnel de traitement ou de conditionnement, ensemble plus ou moins complexe comprenant une pluralité de produits, etc. - destiné à être utilisé à l'intérieur de l'enceinte close.

Par convention, les termes « conteneur » ou « contenant » désignent ce qui, en biopharmacie, est apte et destiné dans son espace intérieur, à contenir, enfermer ou renfermer un certain contenu biopharmaceutique ou le cas échéant plusieurs contenus biopharmaceutiques, de façon statique plus ou moins durable ou pérenne. Un tel contenu biopharmaceutique est typiquement constitué par un ou plusieurs produit(s) biopharmaceutique(s) tel(s) que défini(s) précédemment. De tels conteneurs peuvent être rigides ou souples, à usage unique ou bien réutilisables, de tailles variées et sont par exemple des poches, des gaines, des conteneurs, des récipients, des bioréacteurs ou encore des goulottes à usage biopharmaceutique, cette liste n'étant pas limitative.

Il existe dans le domaine du transfert aseptique de produits biopharmaceutiques le besoin de faire communiquer un conteneur et une enceinte étanche afin d'opérer le transfert de produits biopharmaceutiques sans que l'étanchéité par rapport au milieu extérieur du conteneur et/ou de l'enceinte ne soit rompue et n'entraîne une contamination des produits biopharmaceutiques.

Pour ce faire, il est connu de l'état de la technique la « Porte Biosafe 110 Monolevier » - conforme au préambule de la revendication 1 - qui se rapporte à un dispositif de jonction étanche permettant d'assurer un tel transfert aseptique. Ce dispositif de jonction comprend des moyens stationnaires de bridage temporaire formés par une première couronne annulaire mobile en rotation autour d'un axe géométrique et permettant, via le déplacement d'un bouton poussoir entrainant la rotation de cette première couronne annulaire, d'actionner des éléments de bridage axial permettant de maintenir le conteneur contre la porte de l'enceinte. Le dispositif de jonction comprend également des moyens stationnaires de déverrouillage et de verrouillage, agencés sur la paroi de l'enceinte et permettant de faire passer le conteneur d'une position de verrouillage initial dans laquelle un couvercle amovible obture hermétiquement le conteneur à une position de déverrouillage intermédiaire dans laquelle le couvercle amovible est désolidarisé du conteneur et maintenu hermétiquement contre la porte de l'enceinte pour assurer le transfert aseptique des produis biopharmaceutiques. Ces moyens stationnaires de déverrouillage et de verrouillage sont constitués d'une seconde couronne annulaire dentée, mobile en rotation autour d'un axe géométrique de rotation coïncidant avec l'axe géométrique de la première couronne annulaire et déplaçable grâce à la manipulation d'un levier. Le déplacement du levier dans un sens permet d'entraîner la couronne annulaire dentée dans ce sens et donc de déverrouiller le couvercle amovible du conteneur, tandis que le déplacement du levier dans l'autre sens permet d'entraîner la couronne annulaire dentée en rotation dans le sens inverse et ainsi de verrouiller à nouveau le couvercle amovible contre le conteneur.

Une telle réalisation présente plusieurs inconvénients. En premier lieu, l'opération de bridage du conteneur contre la porte de l'enceinte, d'une part, et les opérations de déverrouillage et de verrouillage du couvercle amovible contre le conteneur, d'autre part, sont assurés par des moyens d'actionnement - le bouton poussoir et le levier - différents et indépendants les uns des autres, ce qui implique des surcoûts de fabrication et ne va pas dans le sens général d'une simplification du dispositif de jonction. Ensuite, la manipulation d'un tel dispositif de jonction, bien que relativement simple, peut parfois entraîner des complications puisque l'opérateur est d'abord conduit à déplacer le bouton poussoir pour assurer le bridage du conteneur contre la porte de l'enceinte, puis ensuite à déplacer le levier dans un premier sens afin de déverrouiller le couvercle amovible du conteneur, et enfin à déplacer ce levier dans un autre sens afin de verrouiller le couvercle amovible contre le conteneur à nouveau. Ces trois actions indépendantes doivent donc être réalisées successivement et à intervalles de temps irréguliers ce qui n'est pas intuitif et peut provoquer des erreurs, volontaires ou involontaires, des opérateurs. Des pertes d'étanchéité peuvent alors se produire, tant au sein de l'enceinte que des conteneurs, et contaminer les produits biopharmaceutiques. En outre, une telle multiplicité de moyens d'actionnement entraîne de nombreuses difficultés quant à l'automatisation de ce type de dispositif de jonction.

Il est également connu de l'état de la technique les documents EP-A1-0 688 020 et EP-A1-1 141 974 se rapportant à une technologie similaire à celle précédemment décrite mais pour laquelle le dispositif de jonction ne présente pas de couronne annulaire. Ainsi, les actionnements des moyens stationnaires de bridage et des moyens stationnaires de déverrouillage/verrouillage sont réalisés différemment et via des mécanismes structurellement et fonctionnellement indépendants les uns des autres.

Outre les inconvénients précédemment mentionnés, cette solution implique de manipuler trois leviers indépendants, agencés sur le pourtour de la porte de l'enceinte pour assurer le verrouillage et le déverrouillage du couvercle amovible du conteneur. Les risques d'erreurs de manipulation sont dont multipliés, comme le sont les zones à risques susceptibles d'engendrer des pertes d'intégrité à l'intérieur de l'enceinte close ou du conteneur.

Il est aussi connu de l'état de la technique le document n° WO-A1-2010/054031 qui se rapporte à un dispositif de jonction étanche présentant des moyens stationnaires de verrouillage/déverrouillage similaires à ceux qui ont été décrits en référence aux documents précédents. Toutefois, ce dispositif de jonction étanche est utilisable avec des conteneurs dotés de moyens embarqués de verrouillage/déverrouillage positionnés directement sur la périphérie de la bride annulaire. Ces moyens embarqués de verrouillage/déverrouillage sont formés par plusieurs pions manipulables, indépendamment les uns des autres et déplaçables de façon réversible d'une position dans laquelle ils sont introduits seulement dans la bride du conteneur - le couvercle amovible étant alors libre - à une position dans laquelle ils sont introduits simultanément dans la bride du conteneur et dans le couvercle amovible - ce dernier étant alors maintenu en position de manière à obturer hermétiquement le conteneur.

Une telle réalisation présente également plusieurs inconvénients. En premier lieu, la présence de plusieurs pions pour assurer le verrouillage du couvercle amovible sur le conteneur et déplaçables indépendamment les uns des autres n'assure pas une fiabilité optimale quant à la perte d'intégrité du dispositif de jonction. En effet, les probabilités sont importantes que la manipulation de ces différents pions, selon des mouvements spécifiques pour le verrouillage et le déverrouillage du couvercle amovible, génèrent des erreurs humaines susceptibles de provoquer un défaut d'isolation à l'intérieur de l'un ou l'autre du conteneur et de l'enceinte. En deuxième lieu, l'utilisation de tels pions déplaçables manuellement provoque une usure accélérée du matériel qui doit donc être remplacé plus régulièrement. En troisième lieu, il convient de souligner que, comme précédemment, l'automatisation d'un tel système serait particulièrement complexe et donc coûteux.

Le document WO2010/054031 divulgue un conteneur destiné à assurer le transfert aseptique d'un produit vers une enceinte. Le conteneur comprend une bride annulaire définissant une ouverture et il est prévu un couvercle amovible.

Le document FR 2 872 446 divulgue un dispositif de transfert étanche à double porte pour assurer un transfert étanche entre une première enceinte étanche, par exemple une cellule de confinement et une seconde enceinte étanche, par exemple une boîte de transfert, comprenant deux portes équipées chacune de moyen de verrouillage pour la verrouiller sur une bride comportant une ouverture centrale et d'un organe d'actionnement pour actionner les moyens de verrouillage, dans lequel les organes d'actionnement sont montés tournant dans les portes et comportent un hublot.

Le document GB 2 218 663 divulgue un système à double couvercle comprenant un premier récipient cylindrique, ouvert à une extrémité, un premier couvercle pour le premier récipient, des moyens définissant un orifice pour un second récipient, et un second couvercle pour le dit orifice, le premier récipient ayant un joint périphérique d'étanchéité des moyens pour le port et le premier couvercle, et le second couvercle ayant un joint périphérique d'étanchéité à l'orifice des moyens et au premier couvercle, dans lequel le premier couvercle comprend un mécanisme de capture d'un premier élément rotatif pour fixer le premier couvercle au premier récipient, le premier récipient comprenant des moyens avec lesquels le mécanisme de capture du premier élément rotatif peut s'engager, le second couvercle incorpore un arbre rotatif s'étendant à travers lui, et il est prévu des moyens pour faire tourner l'arbre, et un mécanisme de capture du second organe rotatif qui peut être actionné par l'arbre pour fixer le premier couvercle au second couvercle, le premier couvercle comportant des moyens avec lesquels le mécanisme de capture du second organe rotatif peut s'engager, le système incorporant enfin un mécanisme à dents de crabotage avec au moins vingt dents qui s'engage lorsque le premier couvercle est adjacent au deuxième couvercle de telle sorte que la rotation de l'arbre entraîne la rotation du premier et du second mécanismes de capture rotatifs simultanément.

Le document GB 2 102 719 divulgue un système pour faire passer des matières dangereuses dans et hors d'une enceinte, telle qu'une boîte à gants, à travers un orifice dans une paroi de l'enceinte. L'orifice est normalement fermé par une porte, qui coopère avec une fermeture d'extrémité amovible sur un récipient ou analogue lorsque celui-ci est présenté à et fixé à l'orifice. Le récipient est fixé en position à l'orifice au moyen d'une bague d'accouplement rotativeUn dispositif de verrouillage assure que la porte ne peut pas être ouverte en l'absence d'un conteneur dans le port et que le conteneur ne peut pas être retiré du port quand la porte est ouverte. En lieu et place du conteneur, on peut utiliser un gant solidaire d'un manchon rigide pour permettre à l'opérateur d'effectuer une fonction de travail au sein de la boîte à gants.

Dans ce contexte, l'invention a donc pour but de proposer un dispositif de jonction étanche exempt de l'une au moins des limitations précédemment évoquées.

Plus particulièrement, l'invention se rapporte à un tel dispositif de jonction étanche permettant d'assurer le transfert de produits biopharmaceutique en limitant le nombre de manipulation à effectuer et les risques de pertes d'étanchéité, susceptibles d'être provoqués par des erreurs de manipulation.

À cet égard, l'invention concerne un dispositif de jonction étanche destiné à assurer le transfert aseptique d'un produit biopharmaceutique entre une enceinte munie d'une porte amovible et un conteneur munie d'un couvercle amovible, comprenant : des moyens stationnaires de bridage temporaire aptes à maintenir le conteneur bridé contre l'enceinte de sorte que le couvercle amovible dudit conteneur se trouve appliqué hermétiquement contre la porte de ladite enceinte ; des moyens stationnaires de déverrouillage aptes à faire passer le conteneur d'une position de verrouillage initial dans laquelle le couvercle amovible obture hermétiquement le conteneur à une position de déverrouillage intermédiaire dans laquelle le couvercle amovible est désolidarisé du conteneur et maintenu hermétiquement contre la porte de l'enceinte de manière à assurer une communication aseptique entre ledit conteneur et ladite enceinte ; des moyens stationnaires de verrouillage aptes à faire passer le conteneur de la position de déverrouillage intermédiaire à une position de verrouillage final dans laquelle ledit couvercle amovible obture à nouveau de façon hermétique le conteneur ; une couronne fonctionnelle annulaire apte à se déplacer par rotation autour d'un axe géométrique de rotation de manière à actionner les moyens stationnaires de déverrouillage et les moyens stationnaires de verrouillage du conteneur. Plus particulièrement, l'invention se caractérise par le fait que les moyens stationnaires de bridage temporaire, les moyens stationnaires de déverrouillage et les moyens stationnaires de verrouillage sont liés mécaniquement à la couronne fonctionnelle annulaire et agencés de sorte que la rotation unidirectionnelle de ladite couronne fonctionnelle annulaire autour de l'axe géométrique de rotation entraîne successivement l'actionnement des moyens stationnaires de bridage temporaire assurant le maintien en position du conteneur contre l'enceinte, puis l'actionnement des moyens stationnaires de déverrouillage assurant le passage en position de déverrouillage intermédiaire du conteneur, puis l'actionnement des moyens stationnaires de verrouillage du conteneur assurant le passage en position de verrouillage final du conteneur, et l'actionnement des moyens stationnaires de bridage temporaire du conteneur assurant la libération du conteneur. Cette réalisation présente plusieurs avantages. En premier lieu, l'utilisation d'une couronne annulaire unidirectionnelle pour activer successivement les moyens stationnaires de bridage temporaire, les moyens stationnaires de déverrouillage et les moyens stationnaires de verrouillage est plus simple et facilite considérablement le travail des opérateurs en rendant le processus d'actionnement plus naturel et intuitif ce qui permet de limiter voir de supprimer complètement toute erreur de manipulation volontaire ou involontaire. Par ailleurs, compte-tenu de cette simplification, il est également possible d'envisager une automatisation du transfert aseptique entre l'enceinte et le conteneur sans qu'il soit nécessaire d'asservir de nombreux organes les uns aux autres. En outre, l'opération d'assemblage du dispositif de jonction sur l'enceinte est également simplifiée puisque, contrairement aux réalisations de l'art antérieur, cette solution intègre implique de regrouper la plupart des contraintes mécaniques sur une seule et même pièce, la couronne annulaire, ce qui évite d'avoir à gérer les jeux fonctionnels correspondant lors du montage du dispositif de jonction étanche sur l'enceinte.

Selon une réalisation, le dispositif de jonction étanche comprenant en outre des moyens stationnaires de blocage/déblocage aptes à empêcher/autoriser l'ouverture de la porte de l'enceinte et liés mécaniquement à la couronne fonctionnelle annulaire de sorte que la rotation unidirectionnelle de la couronne fonctionnelle annulaire autour de l'axe géométrique de rotation entraîne successivement l'actionnement des moyens stationnaires de bridage temporaire assurant le maintien en position du conteneur contre l'enceinte, puis simultanément ou successivement l'actionnement des moyens stationnaires de déverrouillage assurant le passage en position de déverrouillage intermédiaire du conteneur et l'actionnement des moyens stationnaires de blocage/déblocage assurant le déblocage de la porte amovible de l'enceinte, puis l'actionnement des moyens stationnaires de verrouillage du conteneur assurant le passage en position de verrouillage final du conteneur, puis simultanément ou successivement l'actionnement des moyens stationnaires de blocage/déblocage assurant le blocage de la porte amovible de l'enceinte et l'actionnement des moyens stationnaires de verrouillage assurant le passage en position de verrouillage final du conteneur, puis l'actionnement des moyens stationnaires de bridage temporaire afin d'assurer la libération du conteneur vis-à-vis de l'enceinte. Les problèmes de perte d'intégrité sont davantage sécurisés dès lors que les moyens stationnaires de blocage/déblocage sont également actionnés par le mouvement unilatéral de couronne annulaire. En effet, la mise en place d'une telle liaison mécanique entre la couronne fonctionnelle annulaire et ces moyens stationnaires de blocage/déblocage permet de garantir la fermeture de la porte amovible contre l'ouverture de l'enceinte dès lors que le dispositif de jonction étanche n'assure pas le verrouillage initial ou le verrouillage final du conteneur.

Dans ce cas, selon une réalisation, les moyens stationnaires de blocage/déblocage comprennent au moins un agencement fonctionnel stationnaire de blocage/déblocage formé par une portion de couronne fonctionnelle formant engrenage et un organe de blocage susceptible d'être engrainé par la portion de couronne fonctionnelle formant engrenage de sorte que lors de la rotation unidirectionnelle de la couronne fonctionnelle l'organe de blocage passe d'une position bloquée dans laquelle une portion a recouvrement empêche la porte de l'enceinte de s'ouvrir à une position débloquée dans laquelle la portion à recouvrement n'empêche plus la porte de l'enceinte de s'ouvrir. L'utilisation d'une portion à recouvrement entraînée mécaniquement par la couronne annulaire permet de sécuriser le blocage de la porte de l'enceinte en évitant qu'un problème d'asservissement ou de fonctionnement (de capteurs, de vérins, d'automates, etc.) n'engendre une faille de sécurité.

Selon une réalisation, les moyens stationnaires de déverrouillage comprennent au moins un agencement fonctionnel stationnaire de déverrouillage formé par une portion de couronne fonctionnelle formant came radiale à profilé interne et/ou externe et un élément poussoir à déplacement radial coopérant avec la portion de couronne fonctionnelle formant came radiale de sorte que, lors de la rotation unidirectionnelle de la couronne fonctionnelle jusqu'à une position de déverrouillage intermédiaire, l'élément poussoir se déplace et fait passer le conteneur de la position de verrouillage initial à la position de déverrouillage intermédiaire. L'utilisation d'une couronne fonctionnelle formant came radiale à profilé interne ou externe agencés sur la couronne permet de simplifier la gestion du dimensionnement, des déplacements et de l'actionnement temporel des moyens stationnaires de verrouillage initial. L'utilisation d'éléments poussoir combiné à ce système de came radiale est en outre une solution fiable, précise et d'encombrement réduit.

Selon une réalisation, les moyens stationnaires de verrouillage comprennent au moins un agencement fonctionnel stationnaire de verrouillage formé par une portion de couronne fonctionnelle formant came radiale à profilé interne et/ou externe, et un élément poussoir à déplacement radial coopérant avec la portion de couronne fonctionnelle formant came radiale de sorte que, lors de la rotation unidirectionnelle de la couronne fonctionnelle jusqu'à une position de verrouillage final, l'élément poussoir se déplace et fait passer le couvercle amovible du conteneur de la position de déverrouillage intermédiaire à la position de verrouillage final. De la même manière que précédemment, la couronne fonctionnelle formant came radiale à profilé interne ou externe permet de simplifier l'actionnement temporel des moyens stationnaires de déverrouillage et de faciliter la gestion du dimensionnement et des déplacements des moyens stationnaires de verrouillage final.

Dans ce cas, selon une réalisation la portion de couronne fonctionnelle formant came radiale des moyens stationnaires de verrouillage et/ou de déverrouillage est formée par un chemin de guidage agencé dans la couronne fonctionnelle annulaire et l'élément poussoir à déplacement radial desdits moyens stationnaires de verrouillage et/ou de déverrouillage comporte un galet agencé dans le chemin de guidage de sorte que la rotation de la couronne fonctionnelle annulaire génère un déplacement radial du galet qui entraîne le déplacement correspondant d'un téton d'activation. L'utilisation d'un chemin de guidage associé à un galet est simple à fabriquer et permet également d'assurer un déplacement précis, fiable et d'encombrement réduit.

Dans ce cas, selon une réalisation, l'agencement fonctionnel stationnaire de déverrouillage et l'agencement fonctionnel stationnaire de verrouillage sont composé du même élément poussoir à déplacement radial.

Plus particulièrement, selon une réalisation la portion de couronne fonctionnelle formant came radiale de l'agencement fonctionnel stationnaire de verrouillage est agencée dans le prolongement de la portion de couronne fonctionnelle formant came radiale l'agencement fonctionnel stationnaire de déverrouillage eu égard au sens de rotation unidirectionnelle de la couronne fonctionnelle annulaire.

Dans ce cas, selon une réalisation, les portions de couronne formant came radiale de l'agencement fonctionnel stationnaire de verrouillage et de l'agencement fonctionnel stationnaire de déverrouillage sont formées par un chemin de guidage continue agencé dans la couronne fonctionnelle annulaire et l'élément poussoir à déplacement radial comporte un galet agencé dans le chemin de guidage continue de sorte que la rotation de la couronne fonctionnelle annulaire génère un déplacement radial du galet qui entraîne le déplacement correspondant d'un téton d'activation. L'utilisation d'un élément poussoir mis en mouvement par un unique galet, placé dans un unique chemin de guidage continue pour faire fonctionner deux agencements fonctionnel stationnaire de verrouillage et de déverrouillage est une solution simple et naturellement économique qui permet, en outre, de limiter les risques de dégradation prématurée du système et d'erreur de fabrication.

Selon une réalisation alternative, les moyens stationnaires de déverrouillage et les moyens stationnaires de verrouillage peuvent toutefois être structurellement et fonctionnellement distincts et indépendants les uns des autres.

Selon une réalisation, les moyens stationnaires de bridage temporaire comprennent au moins un agencement fonctionnel stationnaire de bridage temporaire réalisés à partir d'une portion de couronne fonctionnelle formant bridage présentant, d'une part, une surface fonctionnelle de bridage axial et, d'autre part, une ouverture d'introduction de moyens embarqués de bridage complémentaire agencés sur une portion de la périphérie extérieure du conteneur. Comme précédemment, utiliser une portion de couronne fonctionnelle formant bridage pour assurer le maintien en position axial du conteneur permet de limiter les coûts de fabrication en évitant l'utilisation d'une pièce distincte de la couronne fonctionnelle annulaire. Par ailleurs, cette solution comportant une surface fonctionnelle de bridage axial et une ouverture d'introduction permet d'indexer la position du conteneur au moment de sa mise en place contre le disposition jonction étanche. En outre, cette solution est sécurisante puisque, dans la mesure où la portion de couronne fonctionnelle formant bridage est mécaniquement liée aux moyens stationnaires de déverrouillage et de verrouillage, il est impossible que la bride du conteneur soit relâcher prématurément.

Dans ce cas, selon une réalisation, la portion de couronne fonctionnelle formant bridage est réalisée sur un pourtour périphérique interne de la couronne fonctionnelle annulaire.

Selon, une réalisation, le dispositif de jonction étanche comprend également des moyens stationnaires de manoeuvre de la porte de l'enceinte aptes à ouvrir et à fermer hermétiquement la porte de l'enceinte.

Dans ce cas de figure, selon une réalisation, les moyens stationnaires de manoeuvre de la porte de l'enceinte sont entraînés mécaniquement par la couronne fonctionnelle annulaire. L'association mécanique des moyens stationnaire de manoeuvre de la porte de l'enceinte sur la couronne fonctionnelle annulaire permet de garantir l'actionnement temporel de la porte et de simplifier, pour les mêmes raisons que précédemment, le dispositif de jonction étanche.

Dans le cas précédent, selon une réalisation alternative, les moyens stationnaires de manoeuvre de la porte de l'enceinte sont entraînés par un moteur de manoeuvre asservis sur le déplacement de la couronne fonctionnelle annulaire.

Selon une réalisation dans laquelle le dispositif de jonction étanche comprend des moyens stationnaires de manoeuvre, il est possible que ces derniers soient aptes à déplacer la porte, d'abord, dans une direction axiale, puis ensuite, dans une direction sensiblement perpendiculaire à la direction axiale de sorte que la porte n'entrave pas le passage du produit biopharmaceutique. De cette façon, la porte de l'enceinte, une fois ouverte, n'encombre pas l'accès au conteneur.

Selon une réalisation, la couronne fonctionnelle annulaire, les moyens stationnaires de bridage temporaire du conteneur, les moyens stationnaires de déverrouillage du conteneur et les moyens stationnaires de verrouillage du conteneur sont positionnés à l'extérieur de l'enceinte. La mise en place de ces éléments à l'extérieur de l'enceinte permet de simplifier les opérations de mise en place et de réparation puisque ces derniers ne se trouvent pas en milieu hermétique à l'intérieur de l'enceinte mais sont au contraire accessible depuis l'extérieur.

Dans ce cas, selon une réalisation, de première part, la portion de couronne fonctionnelle formant engrenage de l'agencement fonctionnel stationnaire de blocage/déblocage est positionnée à l'extérieur de l'enceinte, de deuxième part, l'organe de blocage dudit agencement fonctionnel stationnaire de blocage/déblocage est positionné à l'intérieur de l'enceinte et, de troisième part, ledit organe de blocage est entraîné par un arbre de rotation qui traverse une paroi périphérique de l'enceinte.

Selon une réalisation, les moyens stationnaires de bridage temporaire du conteneur, les moyens stationnaires de déverrouillage du conteneur et les moyens stationnaires de verrouillage du conteneur comprennent, respectivement, n agencement(s) fonctionnel(s) stationnaire(s) de bridage temporaire, n agencement(s) fonctionnel(s) stationnaire(s) de déverrouillage, n agencement(s) fonctionnel(s) stationnaire(s) de verrouillage, avec n supérieur ou égal à 1, de sorte qu'à chaque déplacement en rotation de la couronne fonctionnelle annulaire dans la direction unidirectionnelle se rapportant à 1/n rotation complète corresponde l'actionnement successif des moyens stationnaires de bridage temporaire du conteneur assurant le maintien en position du conteneur contre l'enceinte, des moyens stationnaires de déverrouillage du conteneur assurant le passage en position de déverrouillage intermédiaire du conteneur, des moyens stationnaires de verrouillage du conteneur assurant le passage en position de verrouillage final du conteneur, des moyens stationnaires de bridage temporaire du conteneur assurant la libération dudit conteneur. Le fait de répartir une pluralité d'agencements fonctionnels sur la couronne fonctionnelle annulaire permet de réaliser plusieurs cycles de transferts aseptiques sur la base d'une seule rotation complète et donc de limiter la vitesse de rotation de cette couronne fonctionnelle annulaire en privilégiant la précision de déplacement. En outre, compte-tenu du positionnement relatif de ces agencements fonctionnels, ces derniers sont indexés les uns par rapport aux autres pour mettre la mise en place du conteneur selon plusieurs positions sans que cela n'engendre de difficulté dans le processus de transfert aseptique.

Dans ce cas, selon une réalisation, n peut être égal à 3 ou 4.

Selon un deuxième aspect, l'invention concerne un ensemble comprenant une enceinte et un dispositif de jonction étanche spécialement destinés à être associés à un conteneur de type à usage unique muni d'un couvercle amovible en vue de réaliser le transfert aseptique d'un produit biopharmaceutique entre l'enceinte et le conteneur, dans lequel l'enceinte comprend une paroi périphérique close dans laquelle est aménagée une ouverture hermétiquement obturée par une porte amovible et dans lequel le dispositif de jonction étanche est tel que décrit précédemment.

Dans ce cas, selon une réalisation, l'ensemble comprend, en outre, un conteneur de type à usage unique munie d'un couvercle amovible.

Dans ce cas, selon une réalisation, le conteneur de type à usage unique est spécialement destiné à assurer le transport et le transfert aseptique d'un produit appartenant au domaine biopharmaceutique et comprend : une bride annulaire délimitant une ouverture ; un couvercle amovible apte à obturer hermétiquement l'ouverture de la bride annulaire ; des moyens embarqués de verrouillage/déverrouillage du couvercle amovible sur la bride annulaire ; et une enveloppe périphérique solidaire de la bride annulaire et délimitant un espace intérieur confiné apte à recevoir les produits appartenant au domaine biopharmaceutique. Les moyens embarqués de verrouillage/déverrouillage comprennent au moins un agencement fonctionnel embarqué de verrouillage/déverrouillage formé, d'une part, par un logement traversant formé dans la bride annulaire et un logement borgne formé dans le couvercle amovible et dans le prolongement du logement traversant lorsque le couvercle amovible obture hermétiquement l'ouverture de la bride annulaire et, d'autre part, par une goupille à positionnement radial interne et une goupille à positionnement radial externe susceptibles d'être introduites et de se déplacer à l'intérieur du logement borgne du couvercle amovible et du logement traversant de la bride annulaire. Par ailleurs, le conteneur est apte à se trouver dans une position de verrouillage initial dans laquelle, d'une part, la goupille à positionnement radial interne présente une portion fonctionnelle de verrouillage agencée dans le logement borgne du couvercle amovible et une portion fonctionnelle de verrouillage agencée dans le logement traversant de la bride annulaire de manière à interdire le déplacement relatif du couvercle amovible vis-à-vis de la bride annulaire et, d'autre part, la goupille à positionnement radial externe est au moins partiellement agencée dans le logement traversant de la bride annulaire ; le conteneur est également apte à se trouver dans une position de déverrouillage intermédiaire dans laquelle, d'une part, la goupille à positionnement radial interne est au moins partiellement agencée dans le logement borgne du couvercle amovible et entièrement exclue du logement traversant de la bride annulaire et, d'autre part, la goupille à positionnement radial externe est au moins partiellement agencée dans le logement traversant de la bride annulaire et entièrement exclue du logement borgne du couvercle amovible de sorte à autoriser le déplacement le relatif du couvercle amovible vis-à-vis de la bride annulaire ; le conteneur est, en outre, apte à se trouver dans une position de verrouillage final dans laquelle, d'une part, la goupille à positionnement radial interne est agencée dans le logement borgne du couvercle amovible et, d'autre part, la goupille à positionnement radial externe présente une portion fonctionnelle interne de verrouillage final agencée dans le logement borgne du couvercle amovible et une portion fonctionnelle externe de verrouillage final agencée dans le logement traversant de la bride annulaire de manière à interdire le déplacement relatif du couvercle amovible vis-à-vis de la bride annulaire.

L'invention se rapporte selon un troisième aspect à un procédé de transfert aseptique destiné à assurer le transfert aseptique d'un produit biopharmaceutique entre un conteneur et une enceinte appartenant à un ensemble tel que décrit précédemment, le procédé comprenant des étapes successives consistant à : disposer de l'enceinte, du dispositif de jonction étanche et du conteneur ; positionner le conteneur contre la paroi périphérique de l'enceinte ; générer un bridage axial de la bride annulaire du conteneur contre la paroi périphérique de l'enceinte par rotation unidirectionnelle de la couronne fonctionnelle annulaire ; générer le passage du conteneur de la position de verrouillage initial à la position de déverrouillage intermédiaire par rotation unidirectionnelle de la couronne fonctionnelle annulaire ; ouvrir simultanément la porte amovible de l'enceinte et le couvercle amovible du conteneur, le couvercle amovible étant fixé hermétiquement contre la porte amovible ; transférer de manière aseptique un ou plusieurs(s) produit(s) biopharmaceutique(s) entre le conteneur et l'enceinte ; refermer simultanément la porte amovible de l'enceinte et le couvercle amovible du conteneur, le couvercle amovible étant fixé hermétiquement contre la porte amovible ; générer le passage du conteneur de la position de déverrouillage intermédiaire à la position de verrouillage final par rotation unidirectionnelle de la couronne fonctionnelle annulaire ; générer le débridage axial de la bride annulaire du conteneur vis-à-vis de la paroi périphérique de l'enceinte par rotation unidirectionnelle de la couronne fonctionnelle annulaire.

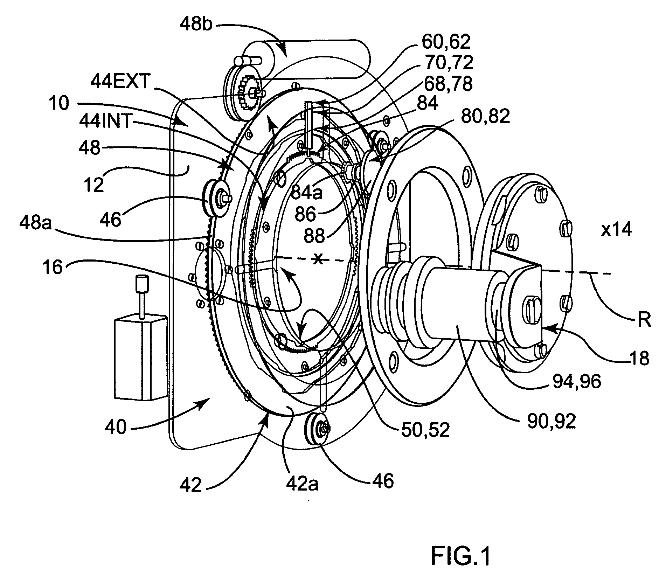

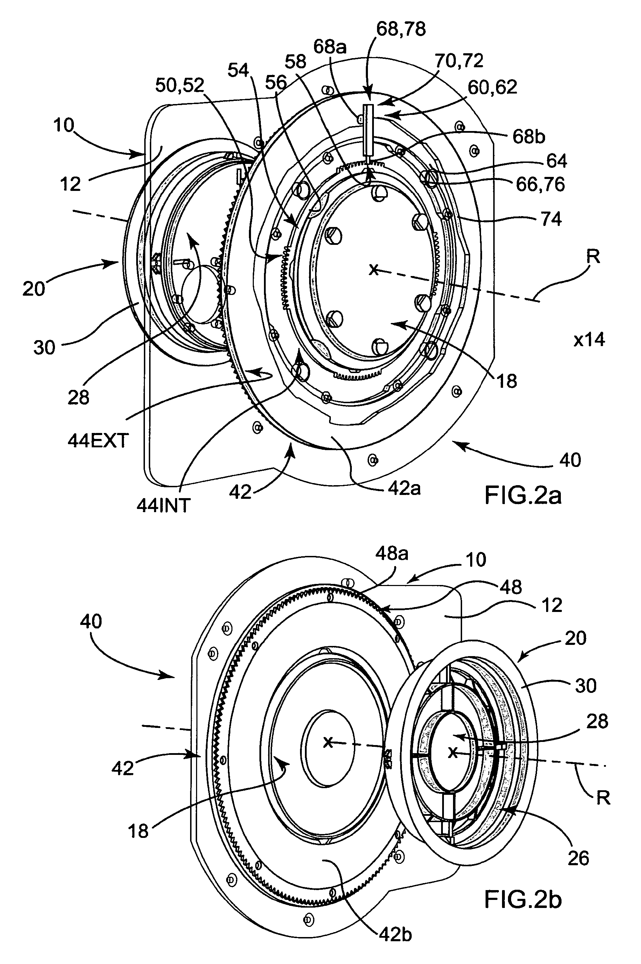

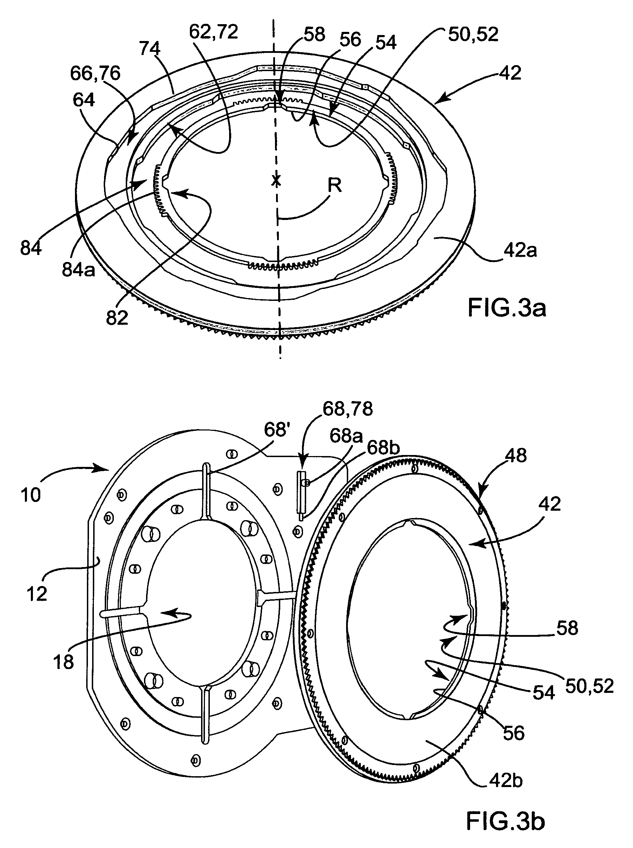

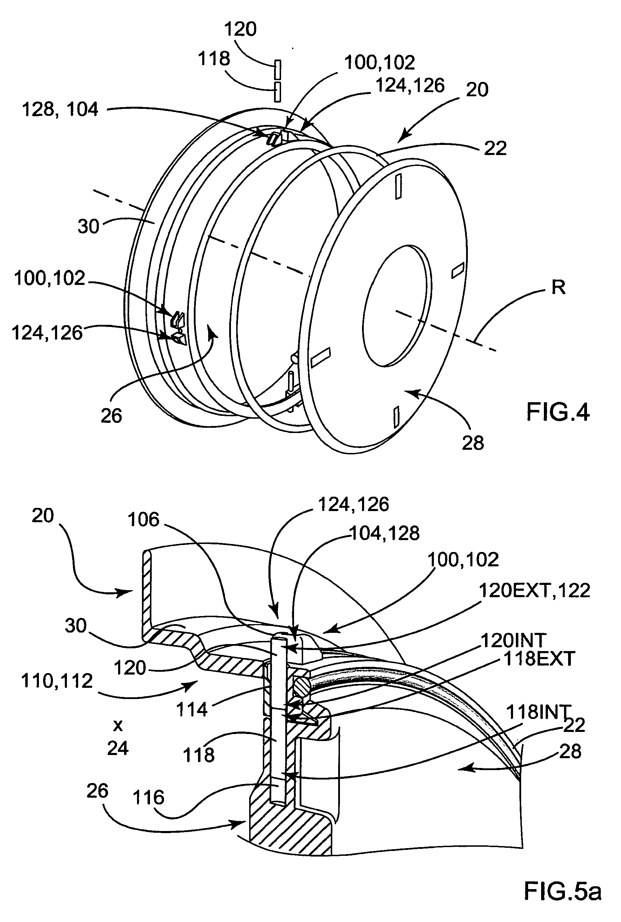

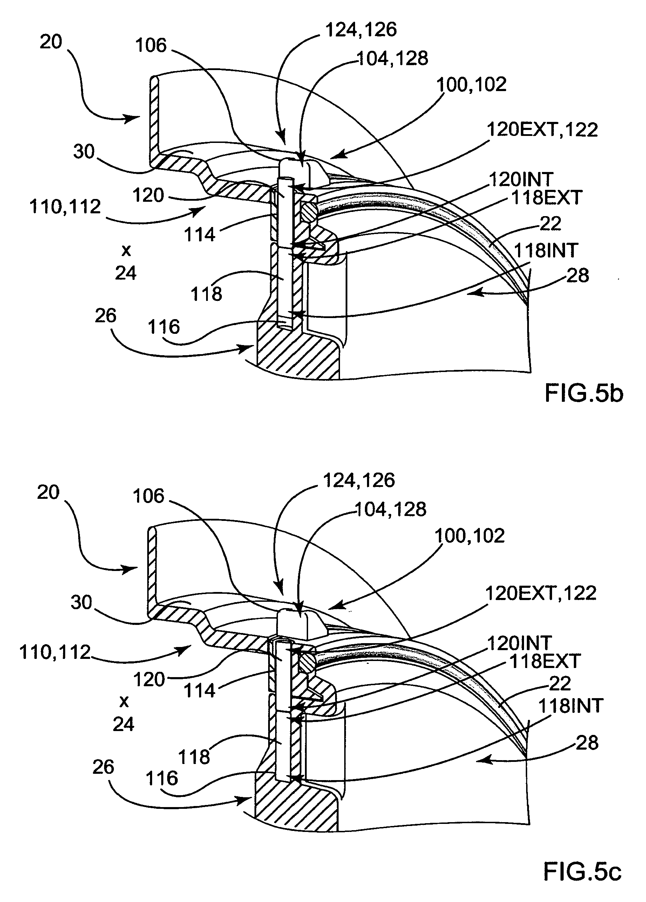

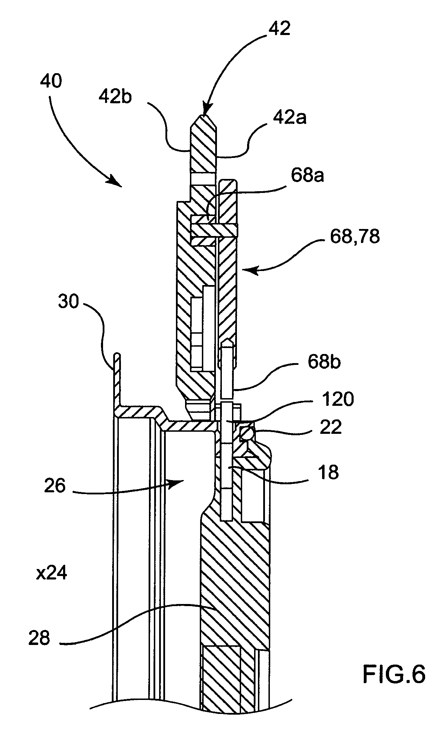

D'autres caractéristiques et avantages de l'invention ressortiront clairement de la description qui en est faite ci-après, à titre indicatif et nullement limitatif, en référence aux dessins annexés, dans lesquels :
▪ La figure 1 est une vue d'ensemble générale, en perspective et en vue éclatée, d'un mode de réalisation d'un dispositif de jonction étanche associé à la paroi périphérique d'une enceinte ;
▪ Les figures 2a et 2b sont deux vues d'ensemble générale, en perspective depuis l'intérieur de l'enceinte et depuis l'extérieur de l'enceinte, du dispositif de jonction étanche de la figure 1 installé sur une portion de la paroi périphérique de l'enceinte et prêt à être associé avec la bride annulaire d'un conteneur selon l'invention ;
▪ Les figures 3a et 3b sont deux vues de détail, en perspective, de la couronne fonctionnelle annulaire appartenant au mode de réalisation du dispositif de jonction de la figure 1 ;
▪ La figure 4 est une vue d'ensemble générale, en perspective et en vue éclatée, d'un mode de réalisation d'un conteneur selon l'invention dans laquelle l'enveloppe périphérique n'est pas représentée ;
▪ La figure 5a est une vue de détail, en coupe transversale, représentant une partie seulement du conteneur de la figure 4 en position de verrouillage initial ;
▪ La figure 5b est une vue de détail, en coupe transversale, représentant une partie seulement du conteneur de la figure 4 en position de déverrouillage intermédiaire ;
▪ La figure 5c est une vue de détail, en coupe transversale, représentant une partie seulement du conteneur de la figure 4 en position de verrouillage final ;
▪ La figure 6 est une représentation de détail, en coupe transversale, d'un ensemble comprenant une enceinte close, un dispositif de jonction étanche et un conteneur selon l'invention associé au dispositif de jonction étanche et en position de verrouillage initiale.

La figure 1 représente une enceinte 10 close comportant une paroi périphérique 12 définissant un espace intérieur 14 confiné et une ouverture circulaire 16 autorisant l'introduction de produits biopharmaceutiques (non représentés) dans l'espace intérieur 14 confiné.

L'enceinte 10 - qui peut être un local ou toute autre installation analogue - est conçue pour être en permanence isolée du milieu extérieur. Ainsi, pour éviter toute perte d'intégrité et conserver un environnement aseptique dans l'espace intérieur 14, l'ouverture annulaire 16 est obturée hermétiquement par une porte amovible 18 positionnée dans l'espace intérieur et capable de passer d'une position fermée dans laquelle l'ouverture circulaire 16 est obstruée à une position ouverte dans laquelle l'ouverture circulaire 16 n'est plus obstruée.

Par convention, les termes « interne » et « externe » qualifient dans la suite de ce document les positions relatives d'objets vis-à-vis de l'axe géométrique de l'ouverture annulaire 16. Ainsi l'objet qualifié d' « interne » ou ayant un « positionnement radial interne » doit-il être considéré comme placé au plus proche de l'axe géométrique de l'ouverture annulaire 16 tandis que l'objet qualifié d' « externe » ou ayant un positionnement radial « externe » doit, comparativement, être considéré comme placé au plus loin de l'axe géométrique de l'ouverture annulaire 16.

Les figures 2a et 2b représentent un conteneur 20 destiné à transporter des produits biopharmaceutiques et prêt à être associé à l'enceinte 10 close pour réaliser un transfert aseptique de produits biopharmaceutiques vers ou depuis cette enceinte 10.

Le conteneur 20 comporte, à cet égard, une enveloppe périphérique (non représentée) définissant un espace intérieur 24 confiné, dans lequel peuvent être introduits les produits biopharmaceutiques. L'enveloppe périphérique comporte également une ouverture annulaire 26 délimitée par une bride annulaire 30 sur laquelle est solidairement fixée l'enveloppe périphérique. Le conteneur 20 comporte, en outre, un couvercle amovible 28 capable d'obturer hermétiquement l'ouverture 24 de la bride annulaire 30 en reposant sur un joint d'étanchéité 22.

Le conteneur 20 peut être réalisé selon différents mode de réalisation et, à cet égard, présenter une enveloppe périphérique souple et destinée à un usage unique ou bien rigide et destinée à être réutilisée après une procédure de décontamination bien connue de l'homme du métier.

Les figures 1, 2a et 2b illustrent un mode de réalisation d'un dispositif de jonction étanche 40.

Ce dispositif de jonction étanche 40 est destiné à permettre le transfert aseptique de produits biopharmaceutiques provenant du conteneur 20 (décrit ultérieurement) vers l'enceinte 10. Le dispositif de jonction étanche 40 a donc pour objet, d'abord, de permettre l'assemblage de l'enceinte 10 et du conteneur 20, ensuite, d'autoriser la mise en communication temporaire de l'espace intérieur 14 de cette enceinte 10 avec l'espace intérieur de ce conteneur 20 pour assurer le transfert de produits biopharmaceutiques de l'un vers l'autre et, enfin, d'assurer la séparation de ladite enceinte 10 et dudit conteneur 20.

Ces étapes successives - englobées par la suite sous la qualification de « transfert aseptique » - doivent être réalisées sans qu'il y ait de communication entre le milieu extérieur, d'une part, et les espaces intérieur 14, 24 de l'enceinte 10 et du conteneur 20, d'autre part.

Pour ce faire, le dispositif de jonction étanche 40 selon le mode de réalisation de la figure 1 présente une partie formée à l'extérieure de l'enceinte 10 et supportée par la paroi périphérique12 - ou par une pièce de support distincte de la paroi périphérique 12 mais fixée à demeure sur elle - en regard de l'ouverture annulaire 16. Plus précisément, le dispositif de jonction étanche 40 comporte une couronne fonctionnelle annulaire 42 dotée d'une face 42a orientée vers la paroi périphérique 12 de l'enceinte 10 et d'une face 42b orientée vers le milieu extérieur.

La couronne fonctionnelle annulaire 42 présente également un pourtour périphérique interne 44_{INT} et un pourtour périphérique externe 44_{EXT} inscrit à l'intérieur de trois galets porteurs 46 eux-mêmes supportés par la face extérieure de la paroi périphérique 12. De cette façon, la couronne fonctionnelle annulaire 42 est positionnée à l'extérieur de l'enceinte 10, ce qui facilite les éventuelles opérations de maintenance. Les trois galets porteurs 46 forment, d'une part, un support pour la couronne fonctionnelle annulaire et permettent, d'autre part, sa rotation autour de l'axe géométrique de l'ouverture annulaire 16. Il convient cependant de souligner que le maintien en position de la couronne annulaire fonctionnelle pourrait être assuré par tout autre élément mécanique analogue (roulement, palier, etc.).

Selon l'exemple de réalisation de la figure 1, la couronne fonctionnelle annulaire 42 qui présente plusieurs portions ayant des fonctions mécaniques distinctes (décrites ultérieurement) est réalisée d'un seul tenant - par usinage, moulage, ou analogue - à partir d'un seul et unique bloc de matière. Cette réalisation présente l'avantage de simplifier la production de la couronne fonctionnelle annulaire, de diminuer dans le même temps les coûts de production et d'assurer un positionnement optimal des surfaces fonctionnelles de la couronne fonctionnelle annulaire 42. Toutefois, il est entendu que la couronne fonctionnelle annulaire pourrait également, selon d'autre modes de réalisation, être formée à partir d'une pluralité de pièces mécaniques distinctes, assemblées les unes autres d'un seul tenant de sorte que la rotation l'une de ces pièces mécaniques autour de l'axe géométrique de rotation R entre la rotation de l'ensemble d'entre elles.

Il convient de signaler que, par convention, les objets dits « stationnaires » font parties du dispositif de jonction étanche 40 et sont donc destinés à être liés et fixés à demeure sur l'enceinte 10. À l'inverse, les objets dits « embarqués » sont destinés à être supportés par les conteneurs 20 et sont donc mobiles avec ces derniers.

La couronne fonctionnelle annulaire 42, plus particulièrement illustré par les figures 3a et 3b, présente une portion stationnaire d'entrainement en rotation 48 agencée à proximité du pourtour périphérique externe de la couronne fonctionnelle annulaire 42 et formée par des dents 48a orientées radialement et susceptibles d'être entraînées par un moteur d'entraînement 48b placés à l'extérieur de l'enceinte 10 ou bien, selon une réalisation alternative, par un levier (non représenté) activé manuellement. Sous l'impulsion de ce moteur d'entraînement 48b, la couronne fonctionnelle annulaire est donc capable d'entrée en rotation autour de l'axe géométrique de rotation R, dans un sens unidirectionnel. Par convention, il sera considéré par la suite que le sens unidirectionnel est le sens horaire. Toutefois, de manière alternative, le sens de rotation de la couronne fonctionnelle annulaire pourrait aussi bien correspondre au sens antihoraire.

Le dispositif de jonction étanche 40 comporte des moyens stationnaires de bridage temporaire 50.

Les moyens stationnaires de bridage temporaire 50 ont pour fonction de maintenir le conteneur 20 bridé contre l'enceinte 10 de sorte que le couvercle amovible 28 de ce conteneur 20 soit appliqué hermétiquement contre la porte amovible 18 de l'enceinte 10.

Ces moyens stationnaires de bridage temporaire 50 sont liés mécaniquement à la couronne fonctionnelle annulaire 42 de sorte que la rotation de cette couronne fonctionnelle annulaire 42 autour de l'axe géométrique de rotation R dans le sens horaire entraîne mécaniquement l'actionnement des moyens stationnaires de bridage temporaire 50.

Selon la réalisation de la figure 1, les moyens stationnaires de bridage temporaire 50 comprennent quatre agencements fonctionnels stationnaires de bridage temporaire 52 régulièrement répartis sur la couronne fonctionnelle annulaire 42. Chacun de ces agencements fonctionnels stationnaires de bridage temporaire 52 est formé à partir d'une portion de couronne fonctionnelle formant bridage 54 appartenant à la couronne fonctionnelle annulaire 42. Chaque portion de couronne fonctionnelle formant bridage 54 présente ainsi, d'une part, une surface fonctionnelle de bridage axial 56 et, d'autre part, une ouverture d'introduction 58 de moyens embarqués de bridage complémentaire agencés sur une portion de la périphérie extérieure du conteneur (décrits ultérieurement).

Dans la réalisation de la figure 1, la surface fonctionnelle de bridage axial 56 est réalisée sur le pourtour périphérique interne 44_{INT} de la couronne fonctionnelle annulaire 42 par un prolongement de matière décalé axialement de la face extérieure de la paroi périphérique 12 de l'enceinte 10 et interrompu régulièrement pour former l'ouverture d'introduction 58. Toutefois, des réalisations alternatives assurant le bridage axial de la bride annulaire 30 du conteneur 20 contre la face extérieure de la paroi périphérique 12 de l'enceinte 10 pourraient également être envisagées.

Le dispositif de jonction étanche 40 comporte des moyens stationnaires de déverrouillage 60.

Les moyens stationnaires de déverrouillage 60 sont destinés à faire passer le conteneur 20 d'une position de verrouillage initial dans laquelle le couvercle amovible 28 obture hermétiquement le conteneur 20 à une position de déverrouillage intermédiaire dans laquelle le couvercle amovible 28 est désolidarisé du conteneur et maintenu hermétiquement contre la porte 18 de l'enceinte 10 de manière à assurer une communication aseptique entre le conteneur 20 et l'enceinte 10.

Comme précédemment, ces moyens stationnaires de de déverrouillage 60 sont liés mécaniquement à la couronne fonctionnelle annulaire 42 de sorte que la rotation de cette couronne fonctionnelle annulaire 42 autour de l'axe géométrique de rotation R et dans le sens horaire entraîne mécaniquement l'actionnement des moyens de déverrouillage 60. Toutefois, compte-tenu de la disposition de ces moyens stationnaires de déverrouillage 60 par rapport aux moyens stationnaires de bridage temporaire 50, l'actionnement des moyens stationnaires de déverrouillage 60 n'intervient qu'à l'issue du bridage axial du conteneur 20 contre l'enceinte 10.

Selon la réalisation de la figure 1, également visible sur les figures 3a et 3b, les moyens stationnaires de déverrouillage 60 comportent plusieurs agencements fonctionnels stationnaires de déverrouillage 62 - en l'occurrence quatre agencements - régulièrement répartis autour de la couronne fonctionnelle annulaire 42.

Chacun de ces agencements fonctionnels stationnaires de déverrouillage 62 comprend, en premier lieu, une portion de couronne fonctionnelle formant came radiale 64 à profilé interne et/ou externe qui appartient à la couronne fonctionnelle annulaire 42. En l'espèce, la portion de couronne fonctionnelle formant came radiale 64 est réalisée à partir d'un chemin de guidage 66 réalisé dans la couronne fonctionnelle annulaire 42 mais cette réalisation n'est pas limitative.

Chacun de ces agencements fonctionnels stationnaires de déverrouillage 62 comprend, en second lieu, un élément poussoir 68 à déplacement radial capable de coopérer avec la portion de couronne fonctionnelle formant came radiale 64. De cette façon, lors de la rotation de la couronne fonctionnelle 42 jusqu'à une position de déverrouillage intermédiaire, le mouvement relatif de la couronne fonctionnelle annulaire 42 vis-à-vis de l'élément poussoir 68 - qui reste fixe en rotation car il est bloqué dans un aménagement complémentaire 68' - provoque le déplacement dudit élément poussoir 68 dans la direction radiale et fait passer le conteneur 20 d'une position de verrouillage initial à une position de déverrouillage intermédiaire (décrites ultérieurement).

Plus particulièrement, selon le mode de réalisation exemplatif et non limitatif de la figure 1, l'élément poussoir 68 qui est fixe en rotation par rapport à la paroi périphérique 12 de l'enceinte 10 comporte, notamment, un galet 68a agencé dans le chemin de guidage 66 et un téton d'activation 68b relié au galet 68a pour que le déplacement de ce dernier dans la direction radiale entraîne un mouvement radial du téton d'activation 68b, jusqu'au passage du conteneur 20 en position de déverrouillage intermédiaire.

Il convient de noter que les termes « déplacement dans la direction radiale » évoqués précédemment doivent être entendus largement et peuvent ainsi correspondre à une direction passant par l'axe géométrique de rotation R ou bien à une direction légèrement inclinée et formant un angle α avec par rapport à la direction radiale de la bride annulaire 30.

Le dispositif de jonction étanche 40 comporte des moyens stationnaires de verrouillage 70.

Les moyens stationnaires de verrouillage 70 sont destinés à faire passer le conteneur 20 de la position de déverrouillage intermédiaire à une position de verrouillage final dans laquelle ledit couvercle amovible 28 obture, à nouveau, le conteneur 20 de façon hermétique.

Comme précédemment, ces moyens stationnaires de verrouillage 70 sont liés mécaniquement à la couronne fonctionnelle annulaire 42 de sorte que la rotation de cette couronne fonctionnelle annulaire 42 autour de l'axe géométrique de rotation R et dans le sens horaire entraîne mécaniquement l'actionnement des moyens stationnaires de verrouillage 70. De la même façon que précédemment, la position relative des moyens stationnaires de verrouillage 70 et des moyens stationnaires de déverrouillage 60 autour de la couronne fonctionnelle annulaire 42 est telle que l'actionnement de ces moyens stationnaires de verrouillage 70 ne peut être que consécutif à l'arrêt des moyens stationnaires de déverrouillage 60.

Selon la réalisation de la figure 1, visible sur les figures 3a et 3b, les moyens stationnaires de verrouillage 70 comportent quatre agencements fonctionnels stationnaires de déverrouillage 72 régulièrement répartis autour de la couronne fonctionnelle annulaire 42.

Chacun de ces agencements fonctionnels stationnaires de déverrouillage 72 comprend, d'abord, une portion de couronne fonctionnelle formant came radiale 74 à profilé interne et/ou externe qui appartient à la couronne fonctionnelle annulaire 42. Comme précédemment, cette portion de couronne fonctionnelle formant came radiale 74 est réalisée à partir d'un chemin de guidage 76 réalisé dans la couronne fonctionnelle annulaire 42 mais cette réalisation n'est pas limitative.

Chacun de ces agencements fonctionnels stationnaires de déverrouillage 72 comprend, ensuite, un élément poussoir 78 à déplacement radial capable de coopérer avec la portion de couronne fonctionnelle formant came radiale 74. De cette façon, lors de la rotation de la couronne fonctionnelle 42 jusqu'à une position de verrouillage final, le mouvement relatif de la couronne fonctionnelle annulaire 42 vis-à-vis de l'élément poussoir 78 - qui reste fixe en rotation car il est bloqué dans un aménagement complémentaire 68' - provoque le déplacement dudit élément poussoir 78 dans la direction radiale et fait passer le conteneur 20 de la position de déverrouillage intermédiaire à une position de verrouillage final (décrite ultérieurement).

Plus particulièrement, selon le mode de réalisation exemplatif et non limitatif de la figure 1, l'élément poussoir 78 qui est fixe en rotation par rapport à la paroi périphérique 12 de l'enceinte 10 est le même que l'élément poussoir 68 appartenant aux moyens fonctionnels stationnaires de déverrouillage 60. Cet élément poussoir 78 comporte ainsi un galet 68a agencé dans le chemin de guidage 76 et un téton d'activation 68b relié au galet 68a pour que le déplacement de ce dernier dans la direction radiale entraîne un mouvement radial du téton d'activation 68b, jusqu'au passage du conteneur 20 en position de déverrouillage intermédiaire.

Il en découle que la portion de couronne fonctionnelle formant came radiale 74 de l'agencement fonctionnel stationnaire de verrouillage 72 est agencée dans le prolongement de la portion de couronne fonctionnelle formant came radiale 64 l'agencement fonctionnel stationnaire de déverrouillage 62 eu égard au sens de rotation unidirectionnelle de la couronne fonctionnelle annulaire 42. À cet égard, les portions de couronne formant came radiale 72, 62 de l'agencement fonctionnel stationnaire de verrouillage 74 et de l'agencement fonctionnel stationnaire de déverrouillage 62 sont formées par un chemin de guidage 66, 76 continue agencé dans la couronne fonctionnelle annulaire 42 et l'élément poussoir 68, 78 à déplacement radial comporte un galet 68a agencé dans le chemin de guidage 66, 76 continue de sorte que la rotation de la couronne fonctionnelle annulaire génère un déplacement radial du galet 68a qui entraîne le déplacement correspondant d'un téton d'activation 38b.

À l'inverse, selon une alternative (non représentée), les moyens stationnaires de déverrouillage 60 et les moyens stationnaires de verrouillage 70 pourraient éventuellement être structurellement et fonctionnellement distincts et indépendants les uns des autres.

Le dispositif de jonction étanche 40 comporte des moyens stationnaires de blocage/déblocage 80.

Ces moyens stationnaires de blocage/déblocage 80 sont destinés à empêcher/autoriser l'ouverture de la porte amovible 18 de l'enceinte 10 et sont reliés mécaniquement à la couronne fonctionnelle annulaire 42 de sorte que la rotation unidirectionnelle de cette couronne fonctionnelle annulaire 42 autour de l'axe géométrique de rotation R et dans le sens horaire entraîne mécaniquement l'actionnement des moyens stationnaire de blocage/déblocage 80. Plus particulièrement, du fait de la position de ces moyens stationnaire de blocage/déblocage 80 sur la couronne fonctionnelle annulaire 42, ces derniers assurent le déblocage de la porte amovible 18 de l'enceinte 10. Selon la réalisation de la figure 1, les moyens stationnaires de blocage/déblocage 80 assurent le déblocage de la porte amovible 18 après que l'actionnement des moyens stationnaires de déverrouillage 60 ait conduit à faire passer le conteneur 20 en position de déverrouillage intermédiaire. De cette façon, l'ouverture de la porte amovible 18 de l'enceinte 10 est retardée *sine die* lorsqu'un problème se produit au cours du déverrouillage intermédiaire du conteneur 20. Cela permet de limiter les problématiques de confinement - nécessairement plus complexe à réparer au sein de l'enceinte 10 - qui pourraient résulter d'une erreur de manipulation du conteneur 20. De façon similaire, ces moyens stationnaires de blocage/déblocage 80 sont agencés sur la couronne fonctionnelle annulaire 42 afin d'assurer le blocage de la porte amovible 18 de l'enceinte 10 avant que les moyens stationnaires de verrouillage 70 aient été activés pour faire passer le conteneur 20 dans la position de verrouillage final.

Selon un autre mode de réalisation, il serait toutefois aussi possible d'agencer les moyens stationnaires de blocage/déblocage 80 sur la couronne fonctionnelle annulaire 42 de sorte que ces derniers assurent le déblocage de la porte amovible 18 simultanément au passage du conteneur 20 en position de déverrouillage intermédiaire, puis le blocage de cette porte amovible 18 simultanément au passage du conteneur 20 en position de verrouillage final.

Enfin, selon un troisième mode de réalisation, il serait également envisageable d'agencer les moyens stationnaires de blocage/déblocage 80 sur la couronne fonctionnelle annulaire 42 de sorte que ces derniers assurent, d'une part, le déblocage de la porte amovible 18 de l'enceinte 10 avant le passage du conteneur 20 en position de déverrouillage intermédiaire et, d'autre part, le blocage de la porte amovible 18 de l'enceinte 10 après le passage du conteneur 20 en position de verrouillage final.

Les moyens stationnaires de blocage/déblocage 80 tel qu'illustrés par la figure 1 comprennent au moins un agencement fonctionnel stationnaire de blocage/déblocage 82 formé par une portion de couronne fonctionnelle formant engrenage 84 appartenant à la couronne fonctionnelle annulaire 42. À ce titre, cette portion de couronne fonctionnelle formant engrenage 84 est positionnée à l'extérieur de l'enceinte 10.

Par ailleurs, selon la réalisation de la figure 1, cette portion de couronne fonctionnelle formant engrenage 84 est crantée de manière à engrainer un arbre de rotation 86, traversant la paroi périphérique 12 de l'enceinte 10 et entraînant lui-même un organe de blocage 88 lors de la rotation unidirectionnelle de la couronne fonctionnelle. Plus particulièrement, l'organe de blocage 88 - qui est se trouve dans l'espace intérieur 14 de l'enceinte 10 - est agencé de manière à pouvoir passer d'une position bloquée dans laquelle une portion à recouvrement 88a empêche la porte amovible 18 de l'enceinte 10 de s'ouvrir à une position débloquée dans laquelle la portion à recouvrement 88a n'empêche plus la porte amovible 18 de s'ouvrir.

Le dispositif de jonction étanche 40 comporte aussi des moyens stationnaires de manoeuvre 90.

Ces moyens stationnaires de manoeuvre 90 sont destinés à ouvrir et refermer hermétiquement la porte amovible 18 de l'enceinte 10.

Pour ce faire, les moyens stationnaires de manoeuvre 90 peuvent, selon un premier mode de réalisation (non représenté), être entraînés mécaniquement par la couronne fonctionnelle annulaire 42. Dans ce cas de figure, les moyens stationnaire de manoeuvre 90 sont reliés mécaniquement à la couronne fonctionnelle annulaire 42 de sorte que la rotation unidirectionnelle de cette couronne fonctionnelle annulaire 42 autour de l'axe géométrique de rotation R et dans le sens horaire entraîne mécaniquement l'actionnement des moyens stationnaires de manoeuvre 90. Plus particulièrement, la position de ces moyens stationnaires de manoeuvre 90 sur la couronne fonctionnelle annulaire 42 permet d'ouvrir la porte amovible 18 de l'enceinte 10 après le passage du conteneur 20 en position de déverrouillage, puis de refermer la porte amovible 18 avant le passage du conteneur 20 en position de verrouillage final.

Selon un second mode de réalisation illustré par la figure 1, les moyens stationnaires de manoeuvre 90 sont entraînés par un moteur de manoeuvre 92 asservi sur le déplacement de la couronne fonctionnelle annulaire 42.

Ces moyens stationnaires de manoeuvre 90 comportent alors un bras rotatif 94 apte à se déplacer en rotation autour d'un axe de rotation horizontal ainsi qu'un bras translatif 96 apte à se déplacer le long d'un axe horizontal. De cette façon, les moyens stationnaires de manoeuvre 90 permettent, grâce au moteur de manoeuvre 92 de déplacer la porte amovible 18 de l'enceinte 10, d'abord, dans une direction axiale, puis ensuite, dans une direction sensiblement perpendiculaire à la direction axiale de sorte que la porte amovible 18 n'entrave pas le passage du produit biopharmaceutique lors de leur transfert aseptique entre l'enceinte 10 et le conteneur 20.

Il convient de noter que, selon l'invention, les moyens stationnaires de bridage temporaire 50, les moyens stationnaires de déverrouillage 60, les moyens stationnaires de verrouillage 70 et éventuellement les moyens de blocage/déblocage 80 et les moyens de manoeuvre 90 sont liés mécaniquement à la couronne fonctionnelle annulaire 42 et agencés de sorte que la rotation unidirectionnelle de ladite couronne fonctionnelle annulaire 42 autour de l'axe géométrique de rotation R et dans le sens horaire entraîne successivement l'actionnement des moyens stationnaires de bridage temporaire 50 pour assurer le maintien en position du conteneur 20 contre l'enceinte 10, puis simultanément ou successivement l'actionnement des moyens stationnaires de déverrouillage 60 assurant le passage en position de déverrouillage intermédiaire du conteneur 20 et l'actionnement des moyens stationnaires de blocage/déblocage 80 assurant le déblocage de la porte amovible 18 de l'enceinte 10, puis simultanément ou successivement l'actionnement des moyens stationnaires de blocage/déblocage 80 assurant le blocage de la porte amovible 18 de l'enceinte 10 et l'actionnement des moyens stationnaires de verrouillage 70 assurant le passage en position de verrouillage final du conteneur 20, et à nouveau l'actionnement des moyens stationnaires de bridage temporaire 50 du conteneur afin de libérer le conteneur 20.

Au surplus, les moyens stationnaires de bridage temporaire 40 sont agencés de manière à assurer le positionnement du conteneur 20 et une indexation par rapport aux autres moyens fonctionnels. De la même façon, l'accumulation de plusieurs agencements fonctionnels cumulés sur la couronne fonctionnelle annulaire 42 permet la mise en oeuvre d'un cycle de manipulation du conteneur 20 en ne réalisant que 1/n tour de rotation. En l'espèce, le mode de réalisation comporte 4 agencements fonctionnels distincts régulièrement répartis sur la couronne fonctionnelle annulaire mais il serait possible d'en inclure moins - à savoir un, deux ou trois - ou bien d'en inclure davantage.

Il convient de signaler que la rotation unidirectionnelle de la couronne fonctionnelle annulaire 42 permet d'assurer l'actionnement des moyens stationnaires de bridage temporaire, de déverrouillage, de verrouillage, de blocage/déblocage et de manoeuvre que cette couronne fonctionnelle annulaire se déplace dans un sens de rotation ou bien dans l'autre.

Il serait, en outre, aussi possible d'intégrer cette couronne fonctionnelle annulaire 42 non pas à l'extérieur de l'enceinte 10 comme illustré par la réalisation de la figure 1 mais directement dans l'espace intérieur 14 de l'enceinte 10, contre la face intérieure de la paroi périphérique 12. Cette réalisation permettrait, notamment, d'éviter l'utilisation de moyens de blocage/déblocage 80 présentant un arbre de rotation 86 traversant la paroi périphérique 12.

La figure 4 représente de manière plus détaillée un mode de réalisation d'un conteneur destiné à être associé au dispositif de jonction étanche selon l'invention.

Toutefois, dans cette illustration, l'enveloppe périphérique du conteneur 20 n'est pas représentée.

Comme indiqué précédemment, ce conteneur 20 est destiné à permettre le transport et le transfert aseptique de produits biopharmaceutiques depuis ou vers une enceinte 10 dotée du dispositif de jonction étanche 40 selon l'invention.

À cet égard, le conteneur 20 comprend la bride annulaire 30 délimitant l'ouverture annulaire 26, le couvercle amovible 28 apte à obturer hermétiquement l'ouverture 26 de la bride annulaire 30 et l'enveloppe périphérique solidaire de la bride annulaire 30 et délimitant l'espace intérieur 24 confiné susceptible de contenir les produits biopharmaceutiques. De cette façon, lorsqu'il est fermé par le couvercle amovible 28 le conteneur 20 est hermétiquement clos, ce qui prévient toute fuite ou toute introduction de matière dans l'espace intérieur 24. Le conteneur 20 peut être rigide ou souple et réutilisable ou à usage unique selon les cas de figure. À titre exemplatif et nullement limitatif, il peut s'agir d'un conteneur 20 de toutes tailles, tel qu'une poche, une gaine, un récipient, un bioréacteur, une goulotte, etc.

Le conteneur 20 comprend également des moyens embarqués de bridage temporaire 100.

Ces moyens embarqués de bridage temporaire 100 sont agencés sur un pourtour périphérie externe de la bride annulaire 30 de manière à permettre le bridage temporaire du conteneur 20 par blocage axial sur la face extérieure de la paroi périphérique 10 de l'enceinte 10 via l'actionnement du dispositif de jonction étanche 40.

Plus particulièrement, selon la réalisation de la figure 4, les moyens embarqués de bridage temporaire 100 comprennent au moins un agencement fonctionnel embarqué de bridage temporaire 102 formé par au moins un ergot 104, de préférence deux ergots 104, présentant une surface fonctionnelle embarquée de bridage axial 106 apte à venir en appui contre la surface fonctionnelle stationnaire de bridage axial 56 du dispositif de jonction étanche 40. Ces ergots 104 susceptibles d'être positionnés entre la face extérieure de la paroi périphérique 12 de l'enceinte 10 et la surface fonctionnelle stationnaire de bridage axial 56 du dispositif de jonction étanche 40 en passant, d'abord, au travers de l'une des ouvertures d'introduction 58 de ce dispositif de jonction étanche 40 permet de bloquer axialement le déplacement du conteneur 20 vis-à-vis de l'enceinte 10.

Lorsque le conteneur 20 est placé bridé contre l'enceinte 10, le couvercle amovible 28 du conteneur 20 est maintenu à demeure - par aimantation ou autre - de manière hermétique contre la porte amovible 108 de l'enceinte 10. De cette façon, l'espace extérieur confiné entre le couvercle amovible 28 et la porte amovible 18 ne peut s'échapper lors du transfert aseptique.

Le conteneur 20 comprend également des moyens embarqués de verrouillage/déverrouillage 110 du couvercle amovible 28 sur la bride annulaire 30.

Ces moyens embarqués de verrouillage/déverrouillage 110 - dont la structure est détaillée ci-après - ont pour fonction d'assurer le maintien du conteneur 20 dans trois positions distinctes. La première position est qualifiée de position de verrouillage initiale car les moyens de verrouillage/déverrouillage 110 interdisent le déplacement relatif du couvercle amovible 28 vis-à-vis de la bride annulaire 30. La deuxième position, qualifiée de position de déverrouillage intermédiaire, est censée intervenir lorsque la bride annulaire 30 du conteneur 20 est bridé axialement contre la paroi périphérique 12 de l'enceinte 10 et autorise le déplacement le relatif du couvercle amovible 28 vis-à-vis de la bride annulaire 30. La troisième position, qualifiée de position de verrouillage final, est supposée intervenir après le transfert aseptique des produits biopharmaceutiques entre le conteneur 20 et l'enceinte 10 et interdit, à nouveau et de manière réversible ou irréversible, le déplacement relatif du couvercle amovible 28 vis-à-vis de la bride annulaire 30.

Pour assurer ce passage de la position de verrouillage initiale à la position de déverrouillage intermédiaire, puis à la position de verrouillage final, les moyens embarqués de verrouillage/déverrouillage 110 selon la réalisation de la figure 4 comportent quatre agencements fonctionnels embarqués de verrouillage/déverrouillage 112 régulièrement répartis autour de la bride annulaire 28 du couvercle 30.

Chaque agencement fonctionnel embarqué de verrouillage/déverrouillage 112 comporte un logement traversant 114 formé dans la bride annulaire 30 ainsi qu'un logement borgne 116 formé dans le couvercle amovible 28 du conteneur 20 et dans le prolongement du logement traversant 114.

Chaque agencement fonctionnel embarqué de verrouillage/déverrouillage 112 comporte également une goupille à positionnement radial interne 118 et une goupille à positionnement radial externe 120. Ces deux goupilles à positionnement radial interne 118 et externe 120 présentent, selon la réalisation de la figure 4, un corps allongé de forme cylindrique leur permettant d'être introduites et de se déplacer à l'intérieur du logement borgne 116 formé dans le couvercle amovible 28, d'une part, et du logement traversant 114 agencé dans la bride annulaire 30, d'autre part. Il convient toutefois de noter que les caractéristiques dimensionnelles et les propriétés de la surface extérieure de ces goupilles à positionnement radial interne 118 et externe 120 sont tels que ces dernières ne peuvent se déplacer à l'intérieur des logements borgne 116 et traversant 114 du seul fait de la gravité. En outre, ces goupilles à positionnement radial interne 118 et externe 120 pourraient présenter un corps de forme différente - par exemple, parallélépipédique - afin de parvenir au même résultat.

Selon la réalisation de la figure 4, logement traversant 114, le logement borgne 116, la goupille à positionnement radial interne 118 et la goupille à positionnement radial externe 120 sont coaxiaux et orientés dans une direction radiale relative à la bride annulaire 30. Plus exactement, selon cette réalisation, ces différents éléments s'étendent selon une direction sensiblement radiale, orientée vers l'axe géométrique de révolution R. Toutefois, selon une réalisation alternative, lesdits logements traversant 114, logement borgne 116, goupille à positionnement radial interne 118 et goupille à positionnement radial externe 120 pourraient également être orientés dans une direction légèrement inclinée et formant un angle α par rapport par rapport à une direction radiale relative à la bride annulaire 30.

D'autre part, selon la réalisation de la figure 4, le logement borgne 116 affleure le logement traversant 114 mais il pourrait également être envisagé de formé un espace intermédiaire entre le logement borgne 116 et le logement traversant 114 sans que cela nuise au confinement et à la manipulation du conteneur 20.

Lorsque le conteneur 20 se trouve dans la position de verrouillage initiale, comme illustré par la figures 5a, la goupille à positionnement radial interne 118 présente une portion fonctionnelle interne de verrouillage initial 118_{INT} agencée dans le logement borgne 116 du couvercle amovible 28 et une portion fonctionnelle externe de verrouillage initial 118_{EXT} agencée dans le logement traversant 114 de la bride annulaire 30. Ainsi, la goupille à positionnement radial interne 118 interdit le déplacement relatif du couvercle amovible 28 vis-à-vis de la bride annulaire 30.

Par ailleurs, dans cette même position de verrouillage initiale, la goupille à positionnement radial externe 120 est au moins partiellement agencée dans le logement traversant 114 de la bride annulaire 30. Selon un premier mode de réalisation (représenté sur la figure 5a), la goupille à positionnement radial externe 120 peut alors présenter une partie extrême fonctionnelle à positionnement radial externe 122 exclue du logement traversant 114. L'accès à cette partie extrême fonctionnelle 122 par les moyens stationnaires de déverrouillage 60 ou de verrouillage 70 est ainsi facilité. Selon un deuxième mode de réalisation (non représenté), cette goupille à positionnement radial externe 120 peut alternativement présenter une partie extrême fonctionnelle à positionnement radial externe 122 affleurant l'extrémité externe débouchant du logement traversant 114. Ainsi, l'accès à cette partie extrême fonctionnelle 122 par les moyens stationnaires de déverrouillage 60 ou de verrouillage 70 reste relativement aisé mais les risques de manipulation inopinée de la goupille à positionnement externe 120 sont néanmoins réduits. Enfin, selon un troisième mode de réalisation (non représenté), ladite goupille à positionnement radial externe 120 peut présenter une partie extrême fonctionnelle à positionnement radial externe 122 entièrement logée à l'intérieur du logement traversant 114. De cette façon, la manipulation de la goupille à positionnement externe 120 est quasiment impossible sans l'utilisation d'un outil spécifique.

Lorsque le conteneur 20 se trouve dans la position de déverrouillage intermédiaire, comme illustré par la figure 5b, la goupille à positionnement radial interne 118 est au moins partiellement agencée dans le logement borgne 116 du couvercle amovible 28 et entièrement exclue du logement traversant 114 de la bride annulaire 30. De cette façon, cette goupille à positionnement radial interne 118 n'interdit plus les déplacement relatif de la bride annulaire 30 et du couvercle amovible 28 du conteneur 20.

Dans cette position de déverrouillage intermédiaire, toujours, la goupille à positionnement radial externe 120 est au moins partiellement agencée dans le logement traversant 114 de la bride annulaire 30 et entièrement exclue du logement borgne 116 du couvercle amovible 28. De cette façon, la goupille à positionnement radial externe 120 n'a pas plus d'influence que la goupille à positionnement radial interne 118 sur le déplacement relatif du couvercle amovible 28 vis-à-vis de la bride annulaire 30 qui peuvent donc librement être désolidarisé pour autoriser le transfert aseptique des produits biopharmaceutiques.

De la même façon que précédemment, la partie extrême fonctionnelle à positionnement radial externe 122 de ladite goupille à positionnement radial externe 120 peut alors soit être exclue du logement traversant 114, soit affleurer l'extrémité externe débouchant du logement traversant 114, soit être logée entièrement à l'intérieur dudit logement traversant 114.

Enfin, pour placer le conteneur 20 dans la position de verrouillage final, comme illustré par la figure 5c, il convient en premier lieu de replacer le couvercle amovible 28 contre la bride annulaire 30 puis de placer l'agencement fonctionnel embarqué de verrouillage/déverrouillage 112 dans la position appropriée.

Plus particulièrement, dans cette position la goupille à positionnement radial interne 118 est entièrement agencée à l'intérieur du logement borgne 116 du couvercle amovible 28 tandis que la goupille à positionnement radial externe 120 présente une portion fonctionnelle interne de verrouillage final 120_{INT} agencée dans le logement borgne 116 du couvercle amovible 28 et une portion fonctionnelle externe de verrouillage final 120_{EXT} agencée dans le logement traversant 114 de la bride annulaire 30. Par conséquent, le logement borgne 116 du couvercle amovible 28 présente une longueur suffisante pour accueillir, d'une part, la goupille à positionnement radial interne 118 et d'autre part, la portion fonctionnelle interne de verrouillage final 120_{INT}. Ainsi, la goupille à positionnement radial externe 120 interdit le déplacement relatif du couvercle amovible 28 vis-à-vis de la bride annulaire 30.

À nouveau, la partie extrême fonctionnelle à positionnement radial externe 122 de ladite goupille à positionnement radial externe 120 peut alors être exclue du logement traversant 114 afin de faciliter l'éventuelle réouverture ultérieure du conteneur 20. Inversement, ladite partie extrême fonctionnelle à positionnement radial externe 122 peut affleurer l'extrémité externe débouchant du logement traversant 114 ou bien être logée entièrement à l'intérieur dudit logement traversant 114 afin de limiter les risque d'ouverture ultérieure du conteneur 20.

Une telle utilisation des goupilles à positionnement radial interne 118 et externe 120 pour placer le conteneur en position de verrouillage initial, de déverrouillage intermédiaire et de verrouillage final permet de n'avoir qu'un logement borgne 116 et un logement traversant 114 à réaliser dans le couvercle amovible 28 et la bride annulaire 30 par agencement fonctionnel embarqué de verrouillage/déverrouillage 112, ce qui limite les risques de contamination lié aux éventuels défauts de production. En outre, la manipulation des moyens de verrouillage/déverrouillage 110 est facilitée dans la mesure où il suffit de déplacer les goupilles à positionnement radial interne 118 et externe 120 dans une seule et unique direction et un seul et même sens pour faire passer le conteneur 20 successivement de la position de verrouillage initial à la position de déverrouillage intermédiaire, puis à la position de verrouillage final. La réalisation et le fonctionnement du dispositif de jonction étanche en sont ainsi simplifiés.

Il convient également de noter que dans la réalisation de la figure 4, les goupilles à positionnement radial interne 118 et externe 120 présentent des longueurs identiques, ce qui permet notamment de simplifier la production et l'assemblage des goupilles à positionnement radial interne 118 et externe 120 sur le conteneur puisqu'il n'est alors pas nécessaire de distinguer les goupilles eu égard à leur dimensions respectives. Toutefois, de manière alternative, ces goupilles à positionnement radial interne 118 et externe 120 pourraient également présenter des longueurs différentes.

Il convient en outre de souligner que le logement traversant 114 et la goupille à positionnement radial externe 120 présentent des longueurs telles que, lorsque les moyens embarqués de verrouillage/déverrouillage 110 sont dans la position de déverrouillage provisoire, la goupille à positionnement radial externe 120 présente une partie extrême fonctionnelle 122 à positionnement radial externe exclue du logement traversant 114. Ce positionnement facilite, notamment, l'opération effectuée par les moyens stationnaires de verrouillage 70 pour faire passer le conteneur 20 de la position de déverrouillage intermédiaire à la position de verrouillage final puisque la goupille à positionnement radial externe 120 est accessible sans que l'élément poussoir à déplacement radial 78 n'ait à se déplacer jusqu'à l'intérieur du logement traversant 114.

En revanche, les longueurs de la goupille à positionnement radial externe 120 et du logement traversant 114 sont telles que, lorsque les moyens embarqués de verrouillage/déverrouillage 110 sont dans la position de verrouillage final, ladite goupille à positionnement radial externe 120 présente une partie extrême fonctionnelle 120 à positionnement radial externe logée à l'intérieure du logement traversant. De cette façon, lorsque le conteneur est placé en position de verrouillage final, la préhension de la goupille à positionnement radial externe 120 est rendu complexe et irréalisable sans un outil de préhension adapté. Cela permet ainsi d'élever le niveau de sécurité se rapportant à l'étanchéité du conteneur 20 en position de verrouillage final.

À cet égard, il pourrait également être envisagé d'intégrer sur la goupille à position radial externe 120 et sur le logement traversant 114 des moyens complémentaires de protection et d'alerte - formés par exemple par des épaulements complémentaires ou tout élément analogue - empêchant le retrait de la goupille à positionnement radial externe 120 hors du logement traversant 114 après la mise en position du conteneur 20 dans la position de verrouillage final et engendrant la destruction d'une partie de la bride annulaire 30 dans l'éventualité d'un tel retrait.

De manière alternative (non représentée), il serait également possible que les longueurs de la goupille à positionnement radial externe 120 et du logement traversant 114 soient telles que, lorsque les moyens embarqués de verrouillage/déverrouillage 110 sont dans la position de verrouillage final, ladite goupille à positionnement radial externe 120 présente une partie extrême fonctionnelle à positionnement radial externe exclue du logement traversant.

Une telle réalisation permettrait, au contraire, de faciliter le retrait de la goupille à positionnement radial externe 120 en dehors du logement traversant 114 et d'obtenir un conteneur réutilisable après sa mise en position de verrouillage final.

Selon la réalisation de la figure 4, le conteneur 20 comporte également des moyens embarqués de cloisonnement et de protection 124.

Les moyens embarqués de cloisonnement et de protection 124 sont agencés sur une portion périphérique externe de la bride annulaire 30 de manière empêcher la manipulation inopinée des moyens embarqués de verrouillage/déverrouillage portés par la bride annulaire 30.

Selon une réalisation, les moyens embarqués de cloisonnement et de protection 124 comprennent quatre agencements fonctionnels embarqués de cloisonnement et de protection 126 disposés autour des quatre logements traversant 114 de manière à faire obstacle à la manipulation inopinée ou volontaire de la goupille à positionnement radial externe 120 lorsque cette dernière n'est pas entièrement insérée dans le logement traversant 118 de la bride annulaire 30.

Chaque agencement fonctionnel embarqué de cloisonnement et de protection 126 est réalisé à partir de deux ergots 128, disposés autour ou de part et d'autre du logement traversant 114. Selon cette réalisation, les ergots 128 présente des dimensions radiales telles que, en position de verrouillage provisoire, la goupille à positionnement radial externe 120 ne s'étend pas au-delà des ergots 128 appartenant moyens de cloisonnement et de protection.

Toutefois, selon une réalisation alternative ou complémentaire, l'agencement fonctionnel embarqué de cloisonnement et de protection 62 pourrait également être réalisé à partir d'un alésage formé dans la bride annulaire 30, orienté radialement et présentant un diamètre supérieur au diamètre du logement traversant 114. Une telle réalisation permet d'empêcher d'accéder à la goupille à positionnement radial externe 120 tout en conservant de l'espace autour de cette goupille à positionnement radial externe 120 afin de ne pas compliquer l'opération de déplacement de cette dernière par les moyens stationnaires de déverrouillage 60 et de verrouillage 70.

Il convient de noter que, selon la réalisation de la figure 4, les ergots 104 formant les moyens embarqués de bridage temporaire 102 et les ergots 128 formant les moyens embarqués de protection et de cloisonnement 124 sont les mêmes. Ainsi, ces ergots 104, 108 présentent, d'une part, une surface fonctionnelle embarquée de bridage axial 56 assurant le bridage du conteneur contre la paroi périphérique 12 de l'enceinte 10 et, d'autre part, une forme assurant la protection et le cloisonnement de la goupille à positionnement radial externe 120 lorsqu'elle n'est que partiellement insérée à l'intérieur du logement traversant 114. Une telle réalisation permet de limiter les coûts de production et de simplifier la bride annulaire 30 en limitant le nombre d'ergots à réaliser sur le pourtour périphérique externe de cette bride annulaire 30.

Cependant, il serait également envisageable d'utiliser des moyens embarqués de bridage temporaire 102 et des moyens embarqués de protection et de cloisonnement 124 structurellement et fonctionnellement indépendants les uns des autres, en utilisant par exemple des ergots 104 pour le bridage axial et d'autres ergots différents pour le cloisonnement et la protection des goupilles à positionnement radial externe 120.

Il convient de signaler comme précédemment que les moyens embarqués de bridage temporaire 100 sont agencés de manière à assurer une indexation de la position du conteneur 20 par rapport aux autres moyens stationnaires appartenant au dispositif de jonction étanche 40.Cette accumulation d'agencements fonctionnels sur la couronne fonctionnelle annulaire 42 permet la mise en oeuvre d'un cycle de manipulation du conteneur 20 en ne réalisant que 1/n tour de rotation avec la couronne fonctionnelle annulaire 42. En l'espèce, le mode de réalisation comporte quatre agencements fonctionnels distincts régulièrement répartis sur la couronne fonctionnelle annulaire 42 mais il serait possible d'en inclure moins - à savoir un, deux ou trois - ou bien d'en inclure davantage.

La mise en oeuvre du procédé de transfert aseptique selon l'invention est maintenant décrite en détail, notamment en référence à la figure 6.

Ce procédé de transfert aseptique consiste, en premier lieu, à disposer d'une enceinte 10 close telle que décrite précédemment et supportant un dispositif de jonction étanche 40 selon l'invention. Ce procédé de transfert aseptique consiste, en deuxième lieu, à disposer d'un conteneur 20 en position de verrouillage initial tel que décrit précédemment.

Le procédé de transfert aseptique implique ensuite de mettre en place le conteneur 20 contre la paroi périphérique 12 de l'enceinte 10 en introduisant les ergots 104 appartenant aux agencement fonctionnels embarqués de bridage temporaire 102 à travers les ouvertures d'introduction 58 appartenant aux moyens stationnaires de bridage temporaire 50.

Ce faisant, la bride annulaire 30 du conteneur 20 vient se positionner contre la paroi périphérique 12 de l'enceinte 10 et le couvercle amovible 28 - au moins partiellement formé de ferrite - est maintenu hermétiquement en position contre la face extérieure de la porte amovible 18 - au moins partiellement aimantée.

Le procédé de transfert aseptique consiste ensuite à générer la rotation de la couronne fonctionnelle annulaire 42 dans le sens horaire afin que les portions de couronne fonctionnelle formant bridage 54 viennent se positionner en regard des ergots 104 formant les moyens embarqués de bridage temporaires et emprisonnent ces ergots 104 entre la paroi périphérique 12 de l'enceinte 10 et les surfaces fonctionnelles de bridage axial 56.

Le conteneur 20 est ainsi maintenu en position par le dispositif de jonction étanche 40 contre la paroi périphérique 12 de l'enceinte 10.

Le procédé de transfert aseptique consiste ensuite à générer la rotation de la couronne fonctionnelle annulaire 42 dans le sens horaire afin d'actionner l'actionnement des moyens stationnaires de déverrouillage 60. Plus particulièrement, la rotation de cette couronne fonctionnelle annulaire 42 dans le sens horaire génère un déplacement de l'élément poussoir à déplacement radial 68 du fait du changement de position radiale du galet 68a dans le chemin de guidage 66. Le téton d'activation 68b s'abaisse alors et vient pousser la goupille à positionnement radial externe 120 et la goupille à positionnement radial interne 118 par la même occasion.

À l'issue de cette opération, la goupille à positionnement radial interne 118 est alors agencée dans le logement borgne 116 du couvercle amovible 28 et entièrement exclue du logement traversant 114 de la bride annulaire 30 et la goupille à positionnement radial externe 120 est agencée dans le logement traversant 114 de la bride annulaire 30 et entièrement exclue du logement borgne 116 du couvercle amovible 28. De ce fait, le conteneur 20 est en position de déverrouillage intermédiaire et le déplacement le relatif du couvercle amovible 28 vis-à-vis de la bride annulaire 30 est possible.

Le procédé de transfert aseptique consiste également, simultanément ou successivement à l'étape qui précède, à générer la rotation de la couronne fonctionnelle annulaire 42 dans le sens horaire afin d'actionner les moyens stationnaires de blocage/déblocage 80 pour libérer la porte amovible 18 de l'enceinte 10.

À cet effet, les portions de couronne fonctionnelle formant engrenage 84 - qui sont entrainées en rotation avec la couronne fonctionnelle annulaire 42 - engrènent l'arbre de rotation 86 et, donc, l'organe de blocage 88 pour que ce dernier ne recouvre plus la face extérieure de la porte amovible 18 de l'enceinte 10. La porte amovible 18 peut alors être manipulée librement.

Le procédé de transfert aseptique consiste alors à entraîner les moyens stationnaires de manoeuvre 90 pour déplacer la porte amovible 18 de l'enceinte 10 et le couvercle amovible du conteneur afin de libérer les ouvertures annulaires 16, 26 de l'enceinte 10 et du conteneur 20.

Pour ce faire, les moyens stationnaires de manoeuvre 90 -qui peuvent être actionnés par la couronne fonctionnelle annulaire 42 ou bien par un moteur de manoeuvre asservis sur la position de cette couronne fonctionnelle annulaire 42 - entraînent en translation horizontale, d'abord, puis en rotation autour d'un axe horizontal, ensuite, la porte amovible 18 de l'enceinte 10.

Le conteneur 20 étant en position de déverrouillage intermédiaire, le couvercle amovible 28 est capable de se déplacer vis-à-vis de la bride annulaire 30 et de suivre les mouvements de la porte amovible 18 de l'enceinte 10 du fait de la force magnétique les reliant l'une à l'autre. Ainsi, l'enceinte 10 et le conteneur 20 sont-ils ouverts l'un sur l'autre tout en étant hermétiquement isolé du milieu extérieur.

Le transfert aseptique des produits biopharmaceutique entre le conteneur 20 et l'enceinte 10 peut alors s'opérer.

Le procédé de transfert aseptique consiste ensuite à entraîner à nouveau les moyens stationnaires de manoeuvre 90 afin de refermer de façon hermétique les ouvertures annulaires 16, 26 de l'enceinte 10 et du conteneur 20.

Comme précédemment, les moyens stationnaires de manoeuvre 90 entraînent alors en rotation autour d'un axe horizontal puis en translation horizontale la porte amovible 18 de l'enceinte 10 ainsi que le couvercle amovible du conteneur 20 pour les remettre dans la même position que précédemment.

L'enceinte 10 est alors à nouveau obturée hermétiquement par la porte amovible 18, tant vis-à-vis de l'extérieur que du conteneur 10. Et, de façon symétrique, le conteneur 20 est obturé hermétiquement par le couvercle amovible, tant vis-à-vis de l'extérieur que de l'enceinte 10.

Le procédé de transfert aseptique consiste alors à générer la rotation de la couronne fonctionnelle annulaire 42 dans le sens horaire afin d'actionner les moyens stationnaires de blocage/déblocage 80 pour bloquer la porte amovible 18 de l'enceinte 10.

À cet effet, les portions de couronne fonctionnelle formant engrenage 84 - qui sont entrainées en rotation avec la couronne fonctionnelle annulaire 42 - engrènent l'arbre de rotation 86 et, donc, l'organe de blocage 88 pour que ce dernier recouvre à nouveau la face extérieure de la porte amovible 18 de l'enceinte 10. La porte amovible 18 ne peut donc plus être manipulée librement.

Le procédé de transfert aseptique consiste, simultanément ou bien successivement à l'étape précédent à générer la rotation de la couronne fonctionnelle annulaire 42 dans le sens horaire afin d'actionner l'actionnement des moyens stationnaires de verrouillage 70. Plus particulièrement, la rotation de cette couronne fonctionnelle annulaire 42 dans le sens horaire génère un déplacement de l'élément poussoir à déplacement radial 78 du fait du changement de position radiale du galet 68a dans le chemin de guidage 76. Le téton d'activation 68b s'abaisse alors et vient pousser la goupille à positionnement radial externe 120 et la goupille à positionnement radial interne 118 par la même occasion.

À l'issue de cette opération, la goupille à positionnement radial interne 118 est agencée dans le logement borgne 116 du couvercle amovible 28 tandis que la goupille à positionnement radial externe 120 présente une portion fonctionnelle interne de verrouillage final 120_{INT} agencée dans le logement borgne 116 du couvercle amovible 28 et une portion fonctionnelle externe de verrouillage final 120_{EXT} agencée dans le logement traversant 114 de la bride annulaire 30. Ainsi le déplacement relatif du couvercle amovible 28 vis-à-vis de la bride annulaire 30 est impossible et le conteneur est alors en position de verrouillage final.

Le procédé de transfert aseptique consiste dès lors à générer la rotation de la couronne fonctionnelle annulaire 42 dans le sens horaire afin que les portions de couronne fonctionnelle formant bridage temporaire 54 se positionnent de sorte que les ouvertures d'introduction 58 soient en regard des ergots 104 formant les moyens embarqués de bridage temporaires et les libèrent.

Le conteneur 20 en position de verrouillage finale peut ainsi être librement retiré du dispositif de jonction étanche 40.

## Revendications

1. Dispositif de jonction (40) étanche destiné à assurer le transfert aseptique d'un produit biopharmaceutique entre une enceinte (10) munie d'une porte amovible (18) et un conteneur (20) munie d'un couvercle amovible (28), comprenant :
des moyens stationnaires de bridage temporaire (50) aptes à maintenir le conteneur (20) bridé contre l'enceinte (10) de sorte que le couvercle amovible (28) dudit conteneur (20) se trouve appliqué hermétiquement contre la porte amovible (18) de ladite enceinte (10) ;
des moyens stationnaires de déverrouillage (60) aptes à faire passer le conteneur (20) d'une position de verrouillage initial dans laquelle le couvercle amovible (28) obture hermétiquement le conteneur (20) à une position de déverrouillage intermédiaire dans laquelle le couvercle amovible (28) est désolidarisé du conteneur (20) et maintenu hermétiquement contre la porte amovible de l'enceinte (10) de manière à assurer une communication aseptique entre ledit conteneur (20) et ladite enceinte (10) ;
des moyens stationnaires de verrouillage (70)aptes à faire passer le conteneur (20) de la position de déverrouillage intermédiaire à une position de verrouillage final dans laquelle ledit couvercle amovible (28) obture à nouveau de façon hermétique le conteneur (20) ;
une couronne fonctionnelle annulaire (42) apte à se déplacer par rotation autour d'un axe géométrique de rotation (R) de manière à actionner les moyens stationnaires de déverrouillage (60) et les moyens stationnaires de verrouillage (70) du conteneur (20) ;
**caractérisé en ce que** les moyens stationnaires de bridage temporaire (50), les moyens stationnaires de déverrouillage (60) et les moyens stationnaires de verrouillage (70) sont liés mécaniquement à la couronne fonctionnelle annulaire (42) et agencés de sorte que la rotation unidirectionnelle de ladite couronne fonctionnelle annulaire (42) autour de l'axe géométrique de rotation (R) entraîne successivement l'actionnement des moyens stationnaires de bridage temporaire (50) assurant le maintien en position du conteneur (20) contre l'enceinte (10), puis l'actionnement des moyens stationnaires de déverrouillage (60) assurant le passage en position de déverrouillage intermédiaire du conteneur (20), puis l'actionnement des moyens stationnaires de verrouillage (70) du conteneur (20) assurant le passage en position de verrouillage final du conteneur (20), et l'actionnement des moyens stationnaires de bridage temporaire (50) du conteneur (20) assurant la libération du conteneur (20).

2. Dispositif de jonction (40) étanche selon la revendication 1, comprenant en outre des moyens stationnaires de blocage/déblocage (80) aptes à empêcher/autoriser l'ouverture de la porte amovible (18) de l'enceinte (10) et liés mécaniquement à la couronne fonctionnelle annulaire (42) de sorte que la rotation unidirectionnelle de la couronne fonctionnelle annulaire (42) autour de l'axe géométrique de rotation (R) entraîne successivement l'actionnement des moyens stationnaires de bridage temporaire (50) assurant le maintien en position du conteneur (20) contre l'enceinte (10), puis simultanément ou successivement l'actionnement des moyens stationnaires de déverrouillage (60) assurant le passage en position de déverrouillage intermédiaire du conteneur (20) et l'actionnement des moyens stationnaires de blocage/déblocage (80) assurant le déblocage de la porte amovible (18) de l'enceinte (10), puis l'actionnement des moyens stationnaires de verrouillage (70) du conteneur (20) assurant le passage en position de verrouillage final du conteneur (20), puis simultanément ou successivement l'actionnement des moyens stationnaires de blocage/déblocage (80) assurant le blocage de la porte amovible (18) de l'enceinte (10) et l'actionnement des moyens stationnaires de verrouillage (70) assurant le passage en position de verrouillage final du conteneur (20), puis l'actionnement des moyens stationnaires de bridage temporaire (50) afin d'assurer la libération du conteneur (20) vis-à-vis de l'enceinte (10).

3. Dispositif de jonction (40) étanche selon la revendication 2, dans lequel les moyens stationnaires de blocage/déblocage (80) comprennent au moins un agencement fonctionnel stationnaire de blocage/déblocage (82) formé par une portion de couronne fonctionnelle formant engrenage (84) et un organe de blocage (88) susceptible d'être engrainé par la portion de couronne fonctionnelle formant engrenage (84) de sorte que, lors de la rotation unidirectionnelle de la couronne fonctionnelle annulaire (42), l'organe de blocage passe d'une position bloquée dans laquelle une portion à recouvrement empêche la porte amovible (18) de l'enceinte (10) de s'ouvrir à une position débloquée dans laquelle la portion à recouvrement n'empêche plus la porte amovible (18) de l'enceinte (10) de s'ouvrir.

4. Dispositif de jonction (40) étanche selon l'une quelconque des revendications 1 à 3, dans lequel les moyens stationnaires de déverrouillage (60) comprennent au moins un agencement fonctionnel stationnaire de déverrouillage (62) formé par une portion de couronne fonctionnelle formant came radiale (64) à profilé interne et/ou externe et un élément poussoir (68) à déplacement radial coopérant avec la portion de couronne fonctionnelle formant came radiale (64) de sorte que, lors de la rotation unidirectionnelle de la couronne fonctionnelle annulaire (42) jusqu'à une position de déverrouillage intermédiaire, l'élément poussoir (68) se déplace et fait passer le conteneur (20) de la position de verrouillage initial à la position de déverrouillage intermédiaire.

5. Dispositif de jonction (40) étanche selon l'une quelconque des revendications 1 à 4, dans lequel les moyens stationnaires de verrouillage (70) comprennent au moins un agencement fonctionnel stationnaire de verrouillage (72) formé par une portion de couronne fonctionnelle formant came radiale (74) à profilé interne et/ou externe, et un élément poussoir (78) à déplacement radial coopérant avec la portion de couronne fonctionnelle formant came radiale (74) de sorte que, lors de la rotation unidirectionnelle de la couronne fonctionnelle annulaire (42) jusqu'à une position de verrouillage final, l'élément poussoir (78) se déplace et fait passer le couvercle amovible (28) du conteneur (20) de la position de déverrouillage intermédiaire à la position de verrouillage final.

6. Dispositif de jonction (40) étanche selon l'une des revendications 4 et 5, dans lequel la portion de couronne fonctionnelle formant came radiale (64, 74) des moyens stationnaires de verrouillage (70) et/ou de déverrouillage (60) est formée par un chemin de guidage (66, 76) agencé dans la couronne fonctionnelle annulaire (42) et l'élément poussoir (68, 78) à déplacement radial desdits moyens stationnaires de verrouillage (70) et/ou de déverrouillage (60) comporte un galet (68a) agencé dans le chemin de guidage (66, 76) de sorte que la rotation de la couronne fonctionnelle annulaire (42) génère un déplacement radial du galet (68a) qui entraîne le déplacement correspondant d'un téton d'activation (68b), **dans lequel de préférence** l'agencement fonctionnel stationnaire de déverrouillage (62) et l'agencement fonctionnel stationnaire de verrouillage (72) sont composé du même élément poussoir (68, 78) à déplacement radial, **dans lequel de préférence** la portion de couronne fonctionnelle annulaire formant came radiale (74) de l'agencement fonctionnel stationnaire de verrouillage (72) est agencée dans le prolongement de la portion de couronne fonctionnelle formant came radiale (64) de l'agencement fonctionnel stationnaire de déverrouillage (62) eu égard au sens de rotation unidirectionnelle de la couronne fonctionnelle annulaire (42), **dans lequel de préférence** les portions de couronne formant came radiale (64, 74) de l'agencement fonctionnel stationnaire de verrouillage (72) et de l'agencement fonctionnel stationnaire de déverrouillage (64) sont formées par un chemin de guidage (66, 76) continue agencé dans la couronne fonctionnelle annulaire (42) et l'élément poussoir (68, 78) à déplacement radial comporte un galet (68a) agencé dans le chemin de guidage (66, 76) continue de sorte que la rotation de la couronne fonctionnelle annulaire (42) génère un déplacement radial du galet qui entraîne le déplacement correspondant d'un téton d'activation (68b), **dans lequel de préférence** les moyens stationnaires de déverrouillage (60) et les moyens stationnaires de verrouillage (70) sont structurellement et fonctionnellement distincts et indépendants les uns des autres.

7. Dispositif de jonction (40) selon l'une quelconque des revendications 1 à 6, dans lequel les moyens stationnaires de bridage temporaire (50) comprennent au moins un agencement fonctionnel stationnaire de bridage temporaire (52) réalisés à partir d'une portion de couronne fonctionnelle formant bridage (54) présentant, d'une part, une surface fonctionnelle de bridage axial (56) et, d'autre part, une ouverture d'introduction (58) de moyens embarqués de bridage complémentaire agencés sur une portion de la périphérie extérieure du conteneur (20), **dans lequel de préférence** la portion de couronne fonctionnelle formant bridage est réalisée sur un pourtour périphérique interne de la couronne fonctionnelle annulaire (42).

8. Dispositif de jonction (40) selon l'une quelconque des revendications 1 à 7, comprenant des moyens stationnaires de manoeuvre de la porte amovible (18) de l'enceinte (10) aptes à ouvrir et à fermer hermétiquement la porte amovible (18) de l'enceinte (10), **dans lequel de préférence** les moyens stationnaires de manoeuvre (90) de la porte amovible (18) de l'enceinte (10) sont entraînés mécaniquement par la couronne fonctionnelle annulaire (42), **dans lequel de préférence** les moyens stationnaires de manoeuvre (90) de la porte amovible (18) de l'enceinte (10) sont entraînés par un moteur de manoeuvre (92) asservis sur le déplacement de la couronne fonctionnelle annulaire (42), **dans lequel de préférence** les moyens stationnaires de manoeuvre (90) de la porte amovible (18) de l'enceinte (10) sont aptes à déplacer la porte amovible de l'enceinte, d'abord, dans une direction axiale, puis ensuite, dans une direction sensiblement perpendiculaire à la direction axiale de sorte que la porte n'entrave pas le passage du produit biopharmaceutique.

9. Dispositif de jonction (40) selon l'une quelconque des revendications 1 à 8, dans lequel la couronne fonctionnelle annulaire (42), les moyens stationnaires de bridage temporaire (50) du conteneur (20), les moyens stationnaires de déverrouillage (60) du conteneur (20) et les moyens stationnaires de verrouillage (70) du conteneur (20) sont positionnés à l'extérieur de l'enceinte (10).

10. Dispositif de jonction (40) selon la revendication 9, en ce qu'elle dépend de la revendication 3, dans lequel, de première part, la portion de couronne fonctionnelle annulaire formant engrenage (84) de l'agencement fonctionnel stationnaire de blocage/déblocage (82) est positionnée à l'extérieur de l'enceinte (10), de deuxième part, l'organe de blocage (88) dudit agencement fonctionnel stationnaire de blocage/déblocage (82) est positionné à l'intérieur de l'enceinte (10) et, de troisième part, ledit organe de blocage (88) est entraîné par un arbre de rotation (86) qui traverse la paroi périphérique (12) de l'enceinte (10).

11. Dispositif de jonction (40) selon l'une quelconque des revendications 1 à 10, dans lequel les moyens stationnaires de bridage temporaire (50) du conteneur (20), les moyens stationnaires de déverrouillage (60) du conteneur (20) et les moyens stationnaires de verrouillage (70) du conteneur (20) comprennent, respectivement, n agencement(s) fonctionnel(s) stationnaire(s) de bridage temporaire (52), n agencement(s) fonctionnel(s) stationnaire(s) de déverrouillage (62), n agencement(s) fonctionnel(s) stationnaire(s) de verrouillage (72), avec n supérieur ou égal à 1, de sorte qu'à chaque déplacement en rotation de la couronne fonctionnelle annulaire (42) dans la direction unidirectionnelle se rapportant à 1/n rotation complète corresponde l'actionnement successif des moyens stationnaires de bridage temporaire (50) du conteneur (20) assurant le maintien en position du conteneur (20) contre l'enceinte (10), des moyens stationnaires de déverrouillage (60) du conteneur (20) assurant le passage en position de déverrouillage intermédiaire du conteneur (20), des moyens stationnaires de verrouillage (70) du conteneur (20) assurant le passage en position de verrouillage final du conteneur (20), des moyens stationnaires de bridage temporaire (50) du conteneur (20) assurant la libération dudit conteneur (20), dans lequel n est de préférence égal à 3 ou 4.

12. Ensemble comprenant une enceinte (10) et un dispositif de jonction (40) étanche spécialement destinés à être associés à un conteneur (20) de type à usage unique muni d'un couvercle amovible (28) en vue de réaliser le transfert aseptique d'un produit biopharmaceutique entre l'enceinte (10) et le conteneur (20), dans lequel l'enceinte (10) comprend une paroi périphérique (12) close dans laquelle est aménagée une ouverture annulaire (16) hermétiquement obturée par une porte amovible (18) et dans lequel le dispositif de jonction (40) étanche est réalisé selon l'une quelconque des revendications 1 à 11.

13. Ensemble selon la revendication 12, comprenant en outre un conteneur (20) de type à usage unique munie d'un couvercle amovible (28).

14. Ensemble selon la revendication 13, dans lequel le conteneur (20) de type à usage unique est spécialement destiné à assurer le transport et le transfert aseptique d'un produit appartenant au domaine biopharmaceutique et comprend :
une bride annulaire (30) délimitant une ouverture (26) ;
un couvercle amovible (28) apte à obturer hermétiquement l'ouverture (26) de la bride annulaire (30) ;
des moyens embarqués de verrouillage/déverrouillage (110) du couvercle amovible (28) sur la bride annulaire (30) ; et
une enveloppe périphérique (22) solidaire de la bride annulaire (30) et délimitant un espace intérieur (24) confiné apte à recevoir les produits appartenant au domaine biopharmaceutique ;
dans lequel les moyens embarqués de verrouillage/déverrouillage (110) comprennent au moins un agencement fonctionnel embarqué de verrouillage/déverrouillage (112) formé, d'une part, par un logement traversant (114) formé dans la bride annulaire (30) et un logement borgne (116) formé dans le couvercle amovible (28) et dans le prolongement du logement traversant (114) lorsque le couvercle amovible (28) obture hermétiquement l'ouverture (26) de la bride annulaire(30) et, d'autre part, par une goupille à positionnement radial interne (118) et une goupille à positionnement radial externe (120) susceptibles d'être introduites et de se déplacer à l'intérieur du logement borgne (116) du couvercle amovible (28) et du logement traversant (114) de la bride annulaire (30) ;
le conteneur (20) étant apte à se trouver dans une position de verrouillage initial dans laquelle, d'une part, la goupille à positionnement radial interne (118) présente une portion fonctionnelle interne de verrouillage (118_{INT}) agencée dans le logement borgne (116) du couvercle amovible (28) et une portion fonctionnelle externe de verrouillage (118_{EXT}) agencée dans le logement traversant (114) de la bride annulaire (30) de manière à interdire le déplacement relatif du couvercle amovible (28) vis-à-vis de la bride annulaire (30) et, d'autre part, la goupille à positionnement radial externe (120) est au moins partiellement agencée dans le logement traversant (114) de la bride annulaire (30) ;
le conteneur (20) étant apte à se trouver dans une position de déverrouillage intermédiaire dans laquelle, d'une part, la goupille à positionnement radial interne (118) est au moins partiellement agencée dans le logement borgne (116) du couvercle amovible (28) et entièrement exclue du logement traversant (114) de la bride annulaire (30) et, d'autre part, la goupille à positionnement radial externe (120) est au moins partiellement agencée dans le logement traversant (114) de la bride annulaire (30) et entièrement exclue du logement borgne (116) du couvercle amovible (28) de sorte à autoriser le déplacement le relatif du couvercle amovible (28) vis-à-vis de la bride annulaire (30) ;
le conteneur (20) étant apte à se trouver dans une position de verrouillage final dans laquelle, d'une part, la goupille à positionnement radial interne (118) est agencée dans le logement borgne (116) du couvercle amovible (28) et, d'autre part, la goupille à positionnement radial externe (120) présente une portion fonctionnelle interne de verrouillage final (120_{INT}) agencée dans le logement borgne (116) du couvercle amovible (28) et une portion fonctionnelle externe de verrouillage final (120_{EXT}) agencée dans le logement traversant (114) de la bride annulaire (30) de manière à interdire le déplacement relatif du couvercle amovible (28) vis-à-vis de la bride annulaire (30).

15. Procédé de transfert aseptique destiné à assurer le transfert aseptique d'un produit biopharmaceutique entre un conteneur (20) et une enceinte (10) appartenant à un ensemble selon l'une des revendications 12 à 14, **caractérisé en ce qu'**il comprend des étapes successives consistant à :
disposer de l'enceinte (10), du dispositif de jonction (40) étanche et du conteneur (20) ;
positionner le conteneur (20) contre la paroi périphérique (12) de l'enceinte (10) ;
générer un bridage axial de la bride annulaire (30) du conteneur (20) contre la paroi périphérique (12) de l'enceinte (10) par rotation unidirectionnelle de la couronne fonctionnelle annulaire (42) ;
générer le passage du conteneur (20) de la position de verrouillage initial à la position de déverrouillage intermédiaire par rotation unidirectionnelle de la couronne fonctionnelle annulaire (42) ;
ouvrir simultanément la porte amovible (18) de l'enceinte (10) et le couvercle amovible (28) du conteneur (20), le couvercle amovible (28) étant fixé hermétiquement contre la porte amovible (18) ;
transférer de manière aseptique un ou plusieurs(s) produit(s) biopharmaceutique(s) entre le conteneur (20) et l'enceinte (10) ;
refermer simultanément la porte amovible (18) de l'enceinte (10) et le couvercle amovible (28) du conteneur (20), le couvercle amovible (28) étant fixé hermétiquement contre la porte amovible (18);
générer le passage du conteneur (20) de la position de déverrouillage intermédiaire à la position de verrouillage final par rotation unidirectionnelle de la couronne fonctionnelle annulaire (42) ;
générer le débridage axial de la bride annulaire (30) du conteneur (20) vis-à-vis de la paroi périphérique (12) de l'enceinte (10) par rotation unidirectionnelle de la couronne fonctionnelle annulaire (42).

## Patentansprüche

1. Dichtes Verbindungsstück (40), das dazu bestimmt ist, den aseptischen Transfer eines biopharmazeutischen Produktes zwischen einem Raum (10), der mit einer abnehmbaren Tür (18) versehen ist, und einem Behälter (20), der mit einem abnehmbaren Deckel (28) versehen ist, zu gewährleisten, umfassend:
- temporär stationäre Flanschmittel (50), die geeignet sind, den angeflanschten Behälter (20) gegen den Raum (10) zu halten, so dass der abnehmbare Deckel (28) des Behälters (20) hermetisch dicht an die abnehmbare Tür (18) des Raums (10) angelegt ist,
- stationäre Entriegelungsmittel (60), die geeignet sind, den Behälter (20) von einer ursprünglichen Verriegelungsposition, in der der abnehmbare Deckel (28) den Behälter (20) hermetisch dicht verschließt, in eine Zwischenentriegelungsposition übergehen zu lassen, in der der abnehmbare Deckel (28) von dem Behälter (20) gelöst und hermetisch dicht an die abnehmbare Tür des Raums (10) gehalten wird, um eine aseptische Verbindung zwischen dem Behälter (20) und dem Raum (10) zu gewährleisten;
- stationäre Verriegelungsmittel (70), die geeignet sind, den Behälter (20) von der Zwischenentriegelungsposition in eine Endverriegelungsposition übergehen zu lassen, in der der abnehmbare Deckel (28) den Behälter (20) wieder hermetisch dicht verschließt;
- einen ringförmigen Funktionskranz (42), der geeignet ist, sich durch Drehung um eine geometrische Drehachse (R) zu bewegen, um die stationären Entriegelungsmittel (60) und die stationären Verriegelungsmittel (70) des Behälters (20) zu betätigen,
**dadurch gekennzeichnet, dass** die temporär stationären Flanschmittel (50), die stationären Entriegelungsmittel (60) und die stationären Verriegelungsmittel (70) mechanisch mit dem ringförmigen Funktionskranz (42) verbunden und derart angeordnet sind, dass die unidirektionale Drehung des ringförmigen Funktionskranzes (42) um die geometrische Drehachse (R) nacheinander zur Betätigung der temporär stationären Flanschmittel (50), die das Halten des Behälters (20) in Position an dem Raum (10) gewährleisten, dann zur Betätigung der stationären Entriegelungsmittel (60), die den Übergang des Behälters (20) in die Zwischenentriegelungsposition gewährleisten, dann zur Betätigung der stationären Verriegelungsmittel (70) des Behälters (20), die den Übergang des Behälters (20) in die Endverriegelungsposition gewährleisten, und zur Betätigung der temporär stationären Flanschmittel (50) des Behälters (20), die die Freigabe des Behälters (20) gewährleisten, führt.

2. Dichtes Verbindungsstück (40) nach Anspruch 1, ferner umfassend stationäre Blockier-/Deblockiermittel (80), die geeignet sind, das Öffnen der abnehmbaren Tür (18) des Raums (10) zu verhindern/gestatten, und die mechanisch mit dem ringförmigen Funktionskranz (42) verbunden sind, so dass die unidirektionale Drehung des ringförmigen Funktionskranzes (42) um die geometrische Drehachse (R) nacheinander zur Betätigung der temporär stationären Flanschmittel (50), die das Halten des Behälters (20) in Position an dem Raum (10) gewährleisten, dann gleichzeitig oder nacheinander zur Betätigung der stationären Entriegelungsmittel (60), die den Übergang des Behälters (20) in die Zwischenentriegelungsposition gewährleisten, und zur Betätigung der stationären Blockier-/Deblockiermittel (80), die das Deblockieren der abnehmbaren Tür (18) des Raums (10) gewährleisten, dann zur Betätigung der stationären Verriegelungsmittel (70) des Behälters (20), die den Übergang des Behälters (20) in die Endverriegelungsposition gewährleisten, dann gleichzeitig oder nacheinander zur Betätigung der stationären Blockier-/Deblockiermittel (80), die das Blockieren der abnehmbaren Tür (18) des Raums (10) gewährleisten, und zur Betätigung der stationären Verriegelungsmittel (70), die den Übergang des Behälters (20) in die Endverriegelungsposition gewährleisten, dann zur Betätigung der temporär stationären Flanschmittel (50) des Behälters (20), die die Freigabe des Behälters (20) gegenüber dem Raum (10) gewährleisten, führt.

3. Dichtes Verbindungsstück (40) nach Anspruch 2, bei dem die stationären Blockier-/Deblockiermittel (80) mindestens eine stationäre Funktionsanordnung zum Blockieren/Deblockieren (82), die von einem Abschnitt eines Funktionskranzes gebildet ist, der ein Getriebe (84) bildet, und ein Blockierelement (88) umfassen, das geeignet ist, den Abschnitt des Funktionskranzes, der ein Getriebe (84) bildet, durch Eingreifen aufzunehmen, so dass bei der unidirektionalen Drehung des ringförmigen Funktionskranzes (42) das Blockierelement von einer blockierten Position, in der ein Überlappungsabschnitt die abnehmbare Tür (18) des Raums (10) daran hindert, sich zu öffnen, in eine deblockierte Position übergeht, in der der Überlappungsabschnitt die abnehmbare Tür (18) des Raums (10) nicht mehr daran hindert, sich zu öffnen.

4. Dichtes Verbindungsstück (40) nach einem der Ansprüche 1 bis 3, bei dem die stationären Entriegelungsmittel (60) mindestens eine stationäre Funktionsanordnung zur Entriegelung (62), die von einem Abschnitt eines Funktionskranzes gebildet ist, der eine radiale Nocke (64) mit Innen-/Außenprofil bildet, und ein Schubelement (68) mit Radialverschiebung umfasst, das mit dem Abschnitt des Funktionskranzes, der eine radiale Nocke (64) bildet, zusammenwirkt, so dass bei der unidirektionalen Drehung des ringförmigen Funktionskranzes (42) bis in eine Zwischenentriegelungsposition sich das Schubelement (68) verschiebt und den Behälter (20) von der ursprünglichen Verriegelungsposition in die Zwischenentriegelungsposition übergehen lässt.

5. Dichtes Verbindungsstück (40) nach einem der Ansprüche 1 bis 4, bei dem die stationären Verriegelungsmittel (70) mindestens eine stationäre Funktionsanordnung zur Verriegelung (72) umfassen, die von einem Abschnitt eines Funktionskranzes, der eine radiale Nocke (74) mit Innen- und/oder Außenprofil bildet, und einem Schubelement (78) mit Radialverschiebung gebildet ist, das mit dem Abschnitt des Funktionskranzes, der die radiale Nocke (74) bildet, zusammenwirkt, so dass bei der unidirektionalen Drehung des ringförmigen Funktionskranzes (42) bis in eine Endverriegelungsposition sich das Schubelement (78) verschiebt und den abnehmbaren Deckel (28) des Behälters (20) von der Zwischenentriegelungsposition in die Endverriegelungsposition übergehen lässt.

6. Dichtes Verbindungsstück (40) nach einem der Ansprüche 4 und 5, bei dem der Abschnitt eines Funktionskranzes der stationären Verriegelungs- (70) und/oder Entriegelungsmittel (60), der eine radiale Nocke (64, 74) bildet, von einem Führungsweg (66, 76) gebildet ist, der in dem ringförmigen Funktionskranz (42) angeordnet ist, und das Schubelement (68, 78) mit Radialverschiebung der stationären Verriegelungs- (70) und/oder Entriegelungsmittel (60) eine Rolle (68a) umfasst, die in dem Führungsweg (66, 76) angeordnet ist, so dass die Drehung des ringförmigen Funktionskranzes (42) eine Radialverschiebung der Rolle (68a) erzeugt, die die entsprechende Verschiebung eines Betätigungszapfens (68b) nach sich zieht, bei dem vorzugsweise die stationäre Entriegelungsfunktionsanordnung (62) und die stationäre Verriegelungsfunktionsanordnung (72) aus demselben Schubelement (68, 78) mit Radialverschiebung bestehen, bei dem vorzugsweise der Abschnitt des ringförmigen Funktionskranzes der stationären Verriegelungsfunktionsanordnung (72), der eine radiale Nocke (74) bildet, in der Verlängerung des Abschnitts des Funktionskranzes der stationären Entriegelungsfunktionsanordnung (62), der eine radiale Nocke (64) bildet, bei Betrachtung der unidirektionalen Drehrichtung des ringförmigen Funktionskranzes (42) angeordnet ist, bei dem vorzugsweise die eine radiale Nocke (64, 74) bildenden Kranzabschnitte der stationären Verriegelungsfunktionsanordnung (72) und der stationären Entriegelungsfunktionsanordnung (64) von einem durchgehenden Führungsweg (66, 76) gebildet sind, der in dem ringförmigen Funktionskranz (42) angeordnet ist, und das Schubelement (68, 78) mit Radialverschiebung eine Rolle (68a) umfasst, die in dem durchgehenden Führungsweg (66, 76) angeordnet ist, so dass die Drehung des ringförmigen Funktionskranzes (42) eine Radialverschiebung der Rolle erzeugt, die die entsprechende Verschiebung eines Betätigungszapfens (68b) nach sich zieht, bei dem vorzugsweise die stationären Entriegelungsmittel (60) und die stationären Verriegelungsmittel (70) strukturell und funktionell getrennt und voneinander unabhängig sind.

7. Verbindungsstück (40) nach einem der Ansprüche 1 bis 6, bei dem die temporär stationären Flanschmittel (50) mindestens eine temporär stationäre Flanschfunktionsanordnung (52) umfassen, die aus einem einen Flansch (54) bildenden Abschnitt eines Funktionskranzes gebildet ist, umfassend einerseits eine axiale Flanschfunktionsfläche (56) und andererseits eine Öffnung (58) zur Einführung von eingebauten komplementären Flanschmitteln, die auf einem Abschnitt der äußeren Peripherie des Behälters (20) angeordnet sind, bei dem vorzugsweise der einen Flansch bildende Abschnitt eines Funktionskranzes auf einem inneren Umfang des ringförmigen Funktionskranzes (42) ausgeführt ist.

8. Verbindungsstück (40) nach einem der Ansprüche 1 bis 7, umfassend stationäre Betätigungsmittel der abnehmbaren Tür (18) des Raums (10), die geeignet sind, die abnehmbare Tür (18) des Raums (10) zu öffnen und hermetisch dicht zu verschließen, bei dem vorzugsweise die stationären Betätigungsmittel (90) der abnehmbaren Tür (18) des Raums (10) mechanisch von dem ringförmigen Funktionskranz (42) angetrieben werden, bei dem vorzugsweise die stationären Betätigungsmittel (90) der abnehmbaren Tür (18) des Raums (10) von einem Betätigungsmotor (92) zur Steuerung der Verschiebung des ringförmigen Funktionskranzes (42) angetrieben werden, bei dem vorzugsweise die stationären Betätigungsmittel (90) der abnehmbaren Tür (18) des Raums (10) geeignet sind, die abnehmbare Tür des Raums zuerst in eine Axialrichtung und dann in eine Richtung im Wesentlichen senkrecht auf die Axialrichtung zu verschieben, so dass die Tür den Durchgang des biopharmazeutischen Produktes nicht behindert.

9. Verbindungsstück (40) nach einem der Ansprüche 1 bis 8, bei dem der ringförmige Funktionskranz (42), die temporär stationären Flanschmittel (50) des Behälters (20), die stationären Entriegelungsmittel (60) des Behälters (20) und die stationären Verriegelungsmittel (70) des Behälters (20) außerhalb des Raums (10) angeordnet sind.

10. Verbindungsstück (40) nach Anspruch 9, insofern er von Anspruch 3 abhängt, bei dem erstens der ein Getriebe (84) bildende Abschnitt des ringförmigen Funktionskranzes der stationären Blockier-/Deblockierfunktionsanordnung (82) außerhalb des Raums (10) angeordnet ist, und zweitens das Blockierelement (88) der stationären Blockier-/Deblockierfunktionsanordnung (82) innerhalb des Raums (10) angeordnet ist, und drittens das Blockierelement (88) durch eine Rotationswelle (86) angetrieben wird, die durch die Umfangswand (12) des Raums (10) hindurchgeht.

11. Verbindungsstück (40) nach einem der Ansprüche 1 bis 10, bei dem die temporär stationären Flanschmittel (50) des Behälters (20), die stationären Entriegelungsmittel (60) des Behälters (20) und die stationären Verriegelungsmittel (70) des Behälters (20) jeweils n stationäre Flanschfunktionsanordnung(en) (52), n stationäre Entriegelungsfunktionsanordnung(en) (62), n stationäre Verriegelungsfunktionsanordnung(en) (72), wobei n größer oder gleich 1 ist, umfassen, so dass jeder Verschiebung des ringförmigen Funktionskranzes (42) in Drehung in die unidirektionale Richtung, die sich auf 1/n vollständige Drehung erstreckt, die aufeinanderfolgende Betätigung der temporär stationären Flanschmittel (50) des Behälters (20), die das Halten des Behälters (20) in Position an dem Raum (10) gewährleisten, der stationären Entriegelungsmittel (60) des Behälters (20), die den Übergang des Behälters (20) in die Zwischenentriegelungsposition gewährleisten, der stationären Verriegelungsmittel (70) des Behälters (20), die den Übergang des Behälters (20) in die Endverriegelungsposition gewährleisten, der temporär stationären Flanschmittel (50) des Behälters (20), die die Freigabe des Behälters (20) gewährleisten, entspricht, wobei n vorzugsweise gleich 3 oder 4 ist.

12. Einheit, umfassend einen Raum (10) und ein dichtes Verbindungsstück (40), die speziell dazu bestimmt sind, mit einem Behälter (20) zur einmaligen Verwendung verbunden zu werden, der mit einem abnehmbaren Deckel (28) versehen ist, um den aseptischen Transfer eines biopharmazeutischen Produktes zwischen dem Raum (10) und dem Behälter (20) durchzuführen, wobei der Raum (10) eine geschlossene Umfangswand (12) umfasst, in der eine ringförmige Öffnung (16) ausgenommen ist, die durch eine abnehmbare Tür (18) hermetisch dicht verschlossen ist, und bei der das dichte Verbindungsstück (40) nach einem der Ansprüche 1 bis 11 hergestellt ist.

13. Einheit nach Anspruch 12, ferner umfassend einen Behälter (20) zur einmaligen Verwendung, der mit einem abnehmbaren Deckel (28) versehen ist.

14. Einheit nach Anspruch 13, bei der der Behälter (20) zur einmaligen Verwendung speziell dazu bestimmt ist, den Transport und den aseptischen Transfer eines Produktes, das dem biopharmazeutischen Bereich angehört, zu gewährleisten, und umfasst:
- einen ringförmigen Flansch (30), der eine Öffnung (26) begrenzt;
- einen abnehmbaren Deckel (28), der geeignet ist, die Öffnung (26) des ringförmigen Flansches (30) hermetisch dicht zu verschließen;
- eingebaute Mittel zur Verriegelung/Entriegelung (110) des abnehmbaren Deckels (28) auf dem ringförmigen Flansch (30); und
- eine Umfangshülle (22), die mit dem ringförmigen Flansch (30) verbunden ist und einen eingegrenzten Innenraum (24) begrenzt, der geeignet ist, die Produkte, die dem biopharmazeutischen Bereich angehören, aufzunehmen;
wobei die eingebauten Verriegelungs-/Entriegelungsmittel (110) mindestens eine eingebaute Funktionsanordnung zur Verriegelung/Entriegelung (112) umfassen, die einerseits von einer Durchgangslagerung (114), die in dem ringförmigen Flansch (30) ausgebildet ist, und einer Scheinlagerung (116), die in dem abnehmbaren Deckel (28) und in der Verlängerung der Durchgangslagerung (114) ausgebildet ist, wenn der abnehmbare Deckel (28) die Öffnung (26) des ringförmigen Flansches (30) hermetisch dicht verschließt, und andererseits von einem Stift zur radial inneren Positionierung (118) und einem Stift zur radial äußeren Positionierung (120) gebildet ist, die geeignet sind, in das Innere der Blindlagerung (116) des abnehmbaren Deckels (28) und der Durchgangslagerung (114) des ringförmigen Flansches (30) eingeführt zu werden und sich in diesen zu verschieben;
wobei der Behälter (20) geeignet ist, sich in einer ursprünglichen Verriegelungsposition zu befinden, in der einerseits der Stift zur radial inneren Positionierung (118) einen inneren Verriegelungsfunktionsabschnitt (118_{INT}), der in der Blindlagerung (116) des abnehmbaren Deckels (28) angeordnet ist, und einen äußeren Verriegelungsfunktionsabschnitt (118_{EXT}) aufweist, der in der Durchgangslagerung (114) des ringförmigen Flansches (30) angeordnet ist, um die relative Verschiebung des abnehmbaren Deckels (28) gegenüber dem ringförmigen Flansch (30) zu untersagen, und andererseits der Stift zur radial äußeren Positionierung (120) zumindest teilweise in der Durchgangslagerung (114) des ringförmigen Flansches (30) angeordnet ist;
wobei der Behälter (20) geeignet ist, sich in einer Zwischenentriegelungsposition zu befinden, in der einerseits der Stift zur radial inneren Positionierung (118) zumindest teilweise in der Blindlagerung (116) des abnehmbaren Deckels (28) angeordnet und zur Gänze aus der Durchgangslagerung (114) des ringförmigen Flansches (30) ausgeschlossen ist, und andererseits der Stift zur radial äußeren Positionierung (120) zumindest teilweise in der Durchgangslagerung (114) des ringförmigen Flansches (30) angeordnet und zur Gänze aus der Blindlagerung (116) des abnehmbaren Deckels (28) ausgeschlossen ist, so dass die relative Verschiebung des abnehmbaren Deckels (28) gegenüber dem ringförmigen Flansch (30) gestattet ist;
wobei der Behälter (20) geeignet ist, sich in einer Endverriegelungsposition zu befinden, in der einerseits der Stift zur radial inneren Positionierung (118) in der Blindlagerung (116) des abnehmbaren Deckels (28) angeordnet ist und andererseits der Stift zur radial äußeren Positionierung (120) einen inneren Funktionsabschnitt zur Endverriegelung (120_{INT}), der in der Blindlagerung (116) des abnehmbaren Deckels (28) angeordnet ist, und einen äußeren Funktionsabschnitt zur Endverriegelung (120_{EXT}) aufweist, der in der Durchgangslagerung (114) des ringförmigen Flansches (30) angeordnet ist, um die relative Verschiebung des abnehmbaren Deckels (28) gegenüber dem ringförmigen Flansch (30) zu untersagen.

15. Verfahren zum aseptischen Transfer, das dazu bestimmt ist, den aseptischen Transfer eines biopharmazeutischen Produkts zwischen einem Behälter (20) und einem Raum (10), die einer Einheit nach einem der Ansprüche 12 bis 14 angehören, zu gewährleisten, **dadurch gekennzeichnet, dass** es nacheinander die folgenden Schritte umfasst, darin bestehend:
- den Raum (10), das dichte Verbindungsstück (40) und den Behälter (20) verfügbar zu haben;
- den Behälter (20) an der Umfangswand (12) des Raums (10) anzuordnen;
- ein axiales Anflanschen des ringförmigen Flansches (30) des Behälters (20) an der Umfangswand (12) des Raums (10) durch unidirektionale Drehung des ringförmigen Funktionskranzes (42) durchzuführen;
- den Übergang des Behälters (20) von der ursprünglichen Verriegelungsposition in die Zwischenentriegelungsposition durch unidirektionale Drehung des ringförmigen Funktionskranzes (42) zu bewirken;
- gleichzeitig die abnehmbare Tür (18) des Raums (10) und den abnehmbaren Deckel (28) des Behälters (20) zu öffnen, wobei der abnehmbare Deckel (28) hermetisch dicht an der abnehmbaren Tür (18) befestigt wird;
- auf aseptische Weise ein oder mehrere biopharmazeutische(s) Produkt(e) zwischen dem Behälter (20) und dem Raum (10) zu transferieren;
- gleichzeitig die abnehmbare Tür (18) des Raums (10) und den abnehmbaren Deckel (28) des Behälters (20) wieder zu schließen, wobei der abnehmbare Deckel (28) hermetisch dicht an der abnehmbaren Tür (18) befestigt wird;
- den Übergang des Behälters (20) von der Zwischenentriegelungsposition in die Endverriegelungsposition durch unidirektionale Drehung des ringförmigen Funktionskranzes (42) zu bewirken;
- das axiale Entflanschen des ringförmigen Flansches (30) des Behälters (20) gegenüber der Umfangswand (12) des Raums (10) durch unidirektionale Drehung des ringförmigen Funktionskranzes (42) durchzuführen.

## Claims

1. Leaktight joining device (40) intended for ensuring the aseptic transfer of a biopharmaceutical product between a chamber (10) equipped with a removable door (18) and a container (20) equipped a removable cover (28), comprising:
stationary temporary clamping means (50) able to keep the container (20) clamped against the chamber (10) so that the removable cover (28) of said container (20) is sealingly held against the removable door (18) of said chamber (10);
stationary unlocking means (60) able to transition the container (20) from an initial locking position where the removable cover (28) seals the container (20) to an intermediate unlocking position where the removable cover (28) is disengaged from the container (20) and is sealingly held against the removable door of the chamber (10) so as to ensure an aseptic communication between said container (20) and said chamber (10);
stationary locking means (70) able to transition the container (20) from the intermediate unlocking position to a final locking position where said removable cover (28) once again seals the container (20);
an annular functional ring gear (42) able to rotate about a geometric axis of rotation (R) so as to actuate the stationary unlocking means (60) and the stationary locking means (70) of the container (20);
**characterized in that** the stationary temporary clamping means (50), stationary unlocking means (60), and stationary locking means (70) are mechanically linked to the annular functional ring gear (42) and are arranged so that the one-way rotation of said annular functional ring gear (42) about the geometric axis of rotation (R) successively causes the actuation of the stationary temporary clamping means (50) which ensures that the container (20) is held in position against the chamber (10), then the actuation of the stationary unlocking means (60) which ensures the transition to the intermediate unlocking position of the container (20), then the actuation of the stationary locking means (70) of the container (20) which ensures the transition to the final locking position of the container (20), and the actuation of the stationary temporary clamping means (50) of the container (20) in order to release the container (20).

2. Leaktight joining device (40) according to claim 1, further comprising stationary retention/release means (80) able to disable/enable the opening of the removable door (18) of the chamber (10) and mechanically linked to the annular functional ring gear (42) such that the one-way rotation of the annular functional ring gear (42) about the geometric axis of rotation (R) successively causes the actuation of the stationary temporary clamping means (50) which ensures that the container (20) is held in position against the chamber (10), then simultaneously or successively the actuation of the stationary unlocking means (60) which ensures the transition to the intermediate unlocking position of the container (20) and the actuation of the stationary retention/release means (80) which ensures the release of the removable door (18) of the chamber (10), then the actuation of the stationary locking means (70) of the container (20) which ensures the transition to the final locking position of the container (20), then simultaneously or successively the actuation of the stationary retention/release means (80) which ensures the retention of the removable door (18) of the chamber (10) and the actuation of the stationary locking means (70) which ensures the transition to the final locking position of the container (20), then the actuation of the stationary temporary clamping means (50) in order to release the container (20) relative to the chamber (10).

3. Leaktight joining device (40) according to claim 2, wherein the stationary retention/release means (80) comprise at least one stationary functional arrangement for retention/release (82) formed by a functional ring portion forming gear teeth (84) and a retention member (88) able to engage with the functional ring portion forming gear teeth (84) such that, during the one-way rotation of the annular functional ring gear (42), the retention member moves from a retention position where a covering portion prevents the removable door (18) of the chamber (10) from opening, to a release position where the covering portion no longer prevents the removable door (18) of the chamber (10) from opening.

4. Leaktight joining device (40) according to one of claims 1 to 3, wherein the stationary unlocking means (60) comprise at least one stationary functional unlocking arrangement (62) formed by a functional ring portion forming an inward- and/or outward-facing radial cam (64) and a radially movable pushing element (68) cooperating with the functional ring portion forming a radial cam (64) so that, during the one-way rotation of the annular functional ring gear (42) to an intermediate unlocking position, the pushing element (68) is moved and causes the container (20) to transition from the initial locking position to the intermediate unlocking position.

5. Leaktight joining device (40) according to one of claims 1 to 4, wherein the stationary locking means (70) comprise at least one stationary functional locking arrangement (72) formed by a functional ring portion forming an inward- and/or outward-facing radial cam (74), and a radially movable pushing element (78) cooperating with the functional ring portion forming a radial cam (74) such that, during the one-way rotation of the annular functional ring gear (42) to a final locking position, the pushing element (78) is moved and causes the removable cover (28) of the container (20) to transition from the intermediate unlocking position to the final locking position.

6. Leaktight joining device (40) according to either of claims 4 or 5, wherein the functional ring portion forming a radial cam (64, 74) of the stationary locking means (70) and/or unlocking means (60) is formed by a guideway (66, 76) arranged in the annular functional ring gear (42), and the radially movable pushing element (68, 78) of said stationary locking means (70) and/or unlocking means (60) comprises a roller (68a) arranged in the guideway (66, 76) so that rotation of the annular functional ring gear (42) generates a radial movement of the roller (68a) which causes the corresponding movement of an activation pin (68b), wherein preferably the stationary functional unlocking arrangement (62) and the stationary functional locking arrangement (72) are composed of the same radially movable pushing element (68, 78), wherein preferably the functional ring portion forming a radial cam (74) of the stationary functional locking arrangement (72) is arranged in the extension of the functional ring portion forming a radial cam (64) of the stationary functional unlocking arrangement (62), considering the one-way direction of rotation of the annular functional ring gear (42), wherein preferably wherein the ring portions forming a radial cam (64, 74) of the stationary functional locking arrangement (72) and of the stationary functional unlocking arrangement (64) are formed by a continuous guideway (66, 76) arranged in the annular functional ring gear (42), and the radially movable pushing element (68, 78) comprises a roller (68a) arranged in the continuous guideway (66, 76) such that the rotation of the annular functional ring gear (42) generates radial movement of the roller which results in the corresponding displacement of an activation pin (68b), wherein preferably the stationary unlocking means (60) and the stationary locking means (70) are structurally and functionally separate and independent of each other.

7. Joining device (40) according to any one of claims 1 to 6, wherein the stationary temporary clamping means (50) comprise at least one stationary functional arrangement for temporary clamping (52) implemented based on a functional clamping ring portion (54) having, on the one hand, a functional surface for axial clamping (56) and, on the other hand, an insertion opening (58) for built-in complementary clamping means arranged on a portion of the outer periphery of the container (20), wherein preferably the functional clamping ring portion is implemented on an inner peripheral edge of the annular functional ring gear (42).

8. Joining device (40) according to any one of claims 1 to 7, comprising stationary operating means for the removable door (18) of the chamber (10), able to open and seal closed the removable door (18) the chamber (10), wherein preferably the stationary operating means (90) for the removable door (18) of the chamber (10) are mechanically driven by the annular functional ring gear (42), wherein preferably the stationary operating means (90) for the removable door (18) of the chamber (10) are driven by a motor (92) controlled by the movements of the annular functional ring gear (42), wherein preferably the stationary operating means (90) for the removable door (18) of the chamber (10) are adapted to move the removable door of the chamber first in an axial direction, then in a direction substantially perpendicular to the axial direction so that the door does not obstruct the passage of the biopharmaceutical product.

9. Joining device (40) according to any one of claims 1 to 8, wherein the annular functional ring gear (42), the stationary temporary clamping means (50) of the container (20), the stationary unlocking means (60) of the container (20), and the stationary locking means (70) of the container (20) are positioned outside the chamber (10).

10. Joining device (40) according to claim 9, as dependent on claim 3, wherein, firstly, the functional ring portion forming gear teeth (84) of the stationary functional arrangement for retention/release (82) is positioned outside the chamber (10), secondly, the retention member (88) of said stationary functional arrangement for retention/release (82) is positioned inside the chamber (10), and thirdly, said retention member (88) is driven by a drive shaft (86) which passes through the peripheral wall (12) of the chamber (10).

11. Joining device (40) according to any one of claims 1 to 10, wherein the stationary temporary clamping means (50) of the container (20), the stationary unlocking means (60) of the container (20), and the stationary locking means (70) of the container (20) respectively comprise n stationary functional arrangement(s) for temporary clamping (52), n stationary functional unlocking arrangement(s) (62), n stationary functional locking arrangement(s) (72), with n greater than or equal to 1, so that with each rotational movement of the annular functional ring gear (42) in the one-way direction corresponding to 1/n complete revolutions there is the corresponding successive actuation of the stationary temporary clamping means (50) of the container (20) to hold the container (20) in position against the chamber (10), of the stationary unlocking means (60) of the container (20) to ensure the transition to the intermediate unlocking position of the container (20), of the stationary locking means (70) of the container (20) to ensure the transition to the final locking position of the container (20), and of the stationary temporary clamping means (50) of the container (20) to ensure the release of said container (20), wherein n is preferably equal to 3 or 4.

12. Assembly comprising a chamber (10) and a leaktight joining device (40) specially intended for association with a single-use container (20) equipped with a removable cover (28) in order to perform the aseptic transfer of a biopharmaceutical product between the chamber (10) and the container (20), wherein the chamber (10) comprises an enclosed peripheral wall (12) having an annular opening (16) that is sealed by a removable door (18), and wherein the leaktight joining device (40) is implemented according to any one of claims 1 to 11.

13. Assembly according to claim 12, further comprising a single-use container (20) equipped with a removable cover (28).

14. Assembly according to claim 13, wherein the single-use container (20) is specially intended for the transport and the aseptic transfer of a product belonging to the biopharmaceutical field and comprises:
an annular flange (30) delimiting an opening (26);
a removable cover (28) adapted for sealing the opening (26) of the annular flange (30);
built-in means (110) for locking/unlocking the removable cover (28) on the annular flange (30); and
a peripheral envelope (22) integral with the annular flange (30) and delimiting an enclosed inside space (24) adapted for receiving products belonging to the biopharmaceutical field;
wherein the bulit-in locking/unlocking means (110) comprise at least one built-in functional locking/unlocking arrangement (112) formed, on the one hand, by a through-housing (114) formed in the annular flange (30) and a blind housing (116) formed in the removable cover (28) and in the extension of the through-housing (114) when the removable cover (28) seals the opening (26) of the annular flange (30), and, on the other hand, by a pin at an inner radial position (118) and a pin at an outer radial position (120) both capable of being introduced and moved within the blind housing (116) of the removable cover (28) and the through-housing (114) of the annular flange (30);
the container (20) being able to be in an initial locking position where, on the one hand, the pin at an inner radial position (118) has an internal functional locking portion (118_{INT}) arranged in the blind housing (116) of the removable cover (28) and an external functional locking portion (118_{EXT}) arranged in the through-housing (114) of the annular flange (30) so as to prevent the relative movement of the removable cover (28) with respect to the annular flange (30), and, on the other hand, the pin at an outer radial position (120) is at least partially arranged in the through-housing (114) of the annular flange (30);
the container (20) being able to be in an intermediate unlocking position where, on the one hand, the pin at an inner radial position (118) is at least partially arranged in the blind housing (116) of the removable cover (28) and is completely outside the through-housing (114) of the annular flange (30), and, on the other hand, the pin at an outer radial position (120) is at least partially arranged in the through-housing (114) of the annular flange (30) and is completely outside the blind housing (116) of the removable cover (28) so as to allow the relative movement of the removable cover (28) with respect to the annular flange (30);
the container (20) being able to be in a final locking position where, on the one hand, the pin at an inner radial position (118) is arranged in the blind housing (116) of the removable cover (28), and, on the other hand, the pin at an outer radial position (120) has an internal functional portion for final locking (120_{INT}) arranged in the blind housing (116) of the removable cover (28) and an external functional portion for final locking (120_{EXT}) arranged in the through-housing (114) of the annular flange (30) so as to prevent the relative movement of the removable cover (28) with respect to the annular flange (30).

15. Aseptic transfer method, intended for the aseptic transfer of a biopharmaceutical product between a container (20) and a chamber (10) which are part of an assembly according to one of claims 12 to 14, **characterized in that** it comprises successive steps consisting of:
having available the chamber (10), the leaktight joining device (40), and the container (20);
positioning the container (20) against the peripheral wall (12) of the chamber (10);
generating an axial clamping of the annular flange (30) of the container (20) against the peripheral wall (12) of the chamber (10) by a one-way rotation of the annular functional ring gear (42);
generating the transition of the container (20) from the initial locking position to the intermediate unlocking position by one-way rotation of the annular functional ring gear (42);
simultaneously opening the removable door (18) of the chamber (10) and the removable cover (28) of the container (20), the removable cover (28) being sealingly attached against the removable gate (18);
aseptically transferring one or more biopharmaceutical product(s) between the container (20) and the chamber (10);
simultaneously closing the removable door (18) of the chamber (10) and the removable cover (28) of the container (20), the removable cover (28) being sealingly attached against the removable door (18);
generating the transition of the container (20) from the intermediate unlocking position to the final locking position by one-way rotation of the annular functional ring gear (42);
generating the axial unclamping of the annular flange (30) of the container (20) with respect to the peripheral wall (12) of the chamber (10) by one-way rotation of the annular functional ring gear (42).
